(19) 

**Europäisches Patentamt**

**European Patent Office**

**Office européen des brevets**

(11) **EP 1 870 478 A1**

(12) **EUROPEAN PATENT APPLICATION**

(43) Date of publication:
**26.12.2007 Bulletin 2007/52**

(51) Int Cl.:
***C12Q 1/68*** (2006.01)

(21) Application number: **07002203.3**

(22) Date of filing: **01.02.2007**

| | |
|---|---|
| (84) Designated Contracting States:<br>**AT BE BG CH CY CZ DE DK EE ES FI FR GB GR HU IE IS IT LI LT LU LV MC NL PL PT RO SE SI SK TR**<br>Designated Extension States:<br>**AL BA HR MK YU** | • **Inoue, Takashi,**<br>**c/o Hitachi, Ltd.**<br>**Chiyoda-ku,**<br>**Tokyo 100-8220 (JP)**<br>• **Taniguchi, Shinichi,**<br>**c/o Hitachi, Ltd.**<br>**Chiyoda-ku,**<br>**Tokyo 100-8220 (JP)** |
| (30) Priority: **20.06.2006 JP 2006170538** | |
| (71) Applicant: **Hitachi, Ltd.**<br>**Tokyo 100-8280 (JP)** | (74) Representative: **Strehl Schübel-Hopf & Partner**<br>**Maximilianstrasse 54**<br>**80538 München (DE)** |
| (72) Inventors:<br>• **Nakahara, Miwako,**<br>**c/o Hitachi, Ltd.**<br>**Chiyoda-ku,**<br>**Tokyo 100-8220 (JP)** | |

(54) **Biosensor element and method for manufacturing the same**

(57)     A biosensor is formed by immobilizing metal particles immobilized on a surface of a carrier and immobilizing probe molecules which are modified with fluorescent molecules on the metal particles. A biomolecule is detected at high sensitivity by use of this biosensor and utilizing fluorescence-quenching and fluorescence-enhancement effects attributable to the metal particle. In this way, it is possible to omit amplification of the biomolecule in a specimen and fluorescence-labeling on the biomolecule when detecting the biomolecule with the biosensor. It is also possible to improve quantitative reliability and repeatability of the biosensor.

FIG. 1

EP 1 870 478 A1

**Description**

CLAIM OF PRIORITY

The present application claims priority from Japanese application JP 2006-170538 filed on June 20, 2006, the content of which is hereby incorporated by reference into this application.

BACKGROUND OF THE INVENTION

1. Field of the Invention

The present invention relates to an element for sensing a biomolecule, a method for manufacturing the same, and method for detecting a biomolecule applying the biosensor element.

2. Description of the Related Art

As decoding of human chromosome DNA has almost been completed in recent years, there has been an increase in the number of researches on "functions created by genes." In the researches it is essential to detect genes and proteins within an organism specifically and comprehensively, and exploitation of techniques for detecting the genes and proteins is therefore in progress worldwide. In the meantime, techniques for identifying pathogens and viruses entering an organism at gene and protein levels have also been studied for a long time, and those techniques are now being put into practical use.

In accordance with these purposes, a biosensor is used as means for detecting a specific biomolecule such as a gene or a protein. The most typical structure of a biosensor is one in which a probe molecule for capturing a biomolecule is immobilized onto a solid surface. A nucleic acid is mainly used as the probe molecule in a case where a nucleic acid is to be captured, while a protein is mainly used as the probe molecule in a case where a protein is to be captured.

An advantage of the biosensor having the probe molecule immobilized onto the substrate is that it is possible to immobilize numerous types of probe molecules on the same substrate by use of a spotting method or an inkjet method. By using this substrate of the biosensor, it is possible to execute a comprehensive analysis on numerous types of biomolecules at a time. Another advantage thereof is that the detection using this biosensor substrate is easy to use as compared to a conventional method of reacting and detecting with a solution system while using a 96-hole plate.

A method using fluorescence detection is known as a method of detecting a biomolecule at high sensitivity. As disclosed in US Patent No. 5,424,186, a fluorescence label is attached to a biomolecule either after amplification of the biomolecule in a specimen or in the course of the amplification, and then the florescence-labeled biomolecule and a biosensor on which a probe molecule is immobilized are brought into a reaction. After the reaction, a light for exciting the fluorescence is irradiated onto the surface of the biosensor. At this time, emission of fluorescence means the fact that the fluorescence-labeled biomolecule in the specimen is captured by the biosensor.

However, this method has the following problems in terms of quantitative reliability and sensitivity. First, fluctuation of fluorescent intensity of the biomolecule to be detected is increased by fluctuation in the amount of modification of the fluorescent molecules labeled on the biomolecule. For example, efficiency of modification varies depending on the type or amount of the biomolecule or on operating staff, and the fluctuation of the modification efficiency deteriorates data repeatability obtained from a biochip. Second, in a case of the currently used biosensor, it is necessary to amplify the biomolecule in the specimen prior to the reaction with the biosensor because of currently achievable sensitivity of the biosensor. Such an amplification process may complicate not only the detection process but also quantitative interpretation of the detection result owing to the fluctuation in the course of the amplification.

To solve the above-mentioned first problem of fluorescence labeling, disclosed is a detection method that does not apply the fluorescence label on a biomolecule to be detected. Japanese Patent Application Publication No. 2004 - 346177 titled as "Imprinted polymer with immobilized gold nanoparticles" discloses a detection method by use of imprinted polymer prepared by burying gold nanoparticles and probe molecules in a substrate without labeling a specimen sample. The absorption of plasmon which is derived from the gold nanoparticles is changed when the probe molecule reacts with the biomolecule in the specimen. Accordingly, a shift in an absorption wavelength is observed in a case where a light is incident on the substrate. An amount of detection is obtained from such an amount of shift. However, the method of detecting the plasmon absorption has a problem of having extremely low sensitivity as compared to the method of detecting the fluorescence.

Meanwhile, a document titled as "Molecular-beacon-based array for sensitive DNA analysis," Analytical Biochemistry 331, p. 216 (2003) discloses an example of detecting a biomolecule without labeling by use of a molecular beacon that includes both of a fluorescent molecule and a quenching molecule as a probe molecule immobilized on a substrate. The basic principle of the molecular beacon is disclosed in US Patent No. 5,925,517, in which the molecular beacon is defined as a molecule that includes the fluorescent molecule and the quenching molecule at both terminals thereof. The fluorescent molecule is usually located close to the quenching molecule, and the fluorescence is quenched by transferring excitation energy from the fluorescent molecule to the quenching molecule. On the other hand, when the beacon reacts with another molecule, the structure of the molecular beacon is changed, and thereby the fluorescence is emitted as a consequence of separation of the fluorescent molecule and the quenching molecule.

Accordingly, when applying the molecular beacon, it is not necessary to put the fluorescence label or the like on the biomolecule for detection because the probe molecule emits the fluorescence by itself. This molecular beacon is generally used as a reagent for detecting the biomolecule in a solution.

However, this molecular beacon has problems of deterioration in fluorescence-quenching efficiency as well as deterioration in fluorescent intensity in a case where the beacon is immobilized on the substrate which is shown in the above-described Analytical Biochemistry 331, p. 216 (2003). This is attributed to the fact that molecular motion is significantly suppressed on the substrate, and it is therefore difficult to transfer the excitation energy from the fluorescent molecule to the quenching molecule while successfully controlling the structure of the molecular beacon. Another reason of the problems is that reaction efficiency of the biomolecule on the substrate is similarly reduced by suppression of the molecular motion, and the fluorescent intensity is thereby reduced.

As a result, it is necessary to enhance quenching efficiency and detection sensitivity in a case of using a biosensor formed by immobilizing the molecular beacon on the substrate.

A document titled as "Single-mismatch detection using gold-quenched fluorescent oligonucleotides," Nature Biotechnology 19, p. 365 (2001) reports that the fluorescence-quenching efficiency in a solution can be improved by use of gold nanoparticles instead of a quenching molecule which is typically used in a molecular beacon. However, this molecular beacon is not immobilized on a substrate, and is therefore not applicable to a biosensor which is capable of performing a comprehensive and highly parallelized analysis or simplified detection.

Next, a method of improving sensitivity of a biosensor has been disclosed as a method of solving the second problem of the fluctuation at the time of amplification. A document titled as "Gold nanoparticle-assisted oligonucleotide immobilization for improved DNA detection," IEE Proc. - Nanobiotechnol. 152, p. 97 (2005) discloses a method of immobilizing gold nanoparticles on a substrate which are modified with single-strand DNA functioning as a probe molecule on a substrate, and then causing the gold nanoparticles to react with a fluorescence-labeled molecule in a specimen. This document reports that the sensitivity of the gold nanoparticles is improved by forming a three-dimensional structure thereof on a surface using the gold nanoparticles. However, it is necessary to label fluorescence on the molecule in the specimen in this case, and consequently this technique is not able to solve the first problem of the fluctuation of labeling.

A document titled as "DNA hybridization assays using metal-enhanced fluorescence", Biochemical and Biophysical Research Communications 306 (2003) discloses a method of utilizing enhancement of fluorescence in order to improve sensitivity. In this document, silver nanoparticles modified with single-strand DNA functioning as a probe molecule is immobilized on a substrate, and the silver nanoparticles is caused to react with fluorescence-labeled molecules in a specimen. When fluorescence excitation light is irradiated to detect a reacting amount, the fluorescence is enhanced by a localized plasmon resonance effect attributable to the silver nanoparticle. The document reports that it is possible to improve the sensitivity by using this phenomenon.

Fluorescence-enhancement effects by metal nanoparticles are described in detail in a document titled as "Radiative Decay Engineering: Biophysical and Biomedical Applications," Analytical Biochemistry 298, p. 1 (2001). However, since the device introduced in Biochemical and Biophysical Research Communications 306 (2003), which is configured to immobilize the DNA on the silver nanoparticle, requires fluorescence labeling on the molecule in the specimen. Accordingly, this technique described in this document cannot solve the first problem of the fluctuation of labeling.

Meanwhile, US 2005/0048546 A1 discloses a method of improving detection sensitivity by utilizing a fluorescence-enhancement effect while applying metal nanoparticles as quenching molecules in molecular beacons. However, this method is also premised on mixing free molecular beacons, which are not immobilized on a surface or the like, in a solution. Accordingly, this method is not applicable as a biosensor which can perform a comprehensive and highly parallelized analysis or simplified detection.

## SUMMARY OF THE INVENTION

A first object of the present invention is to detect a biomolecule for detection without labeling. A second object of the present invention is to detect a biomolecule for detection at high sensitivity without amplification. Highly accurate detection of a biomolecule is achieved in a comprehensive and simplified manner by use of a biosensor element that can attain the foregoing two objects.

The present invention detects a biomolecule without labeling by use of a biosensor element formed by immobilizing metal particles on a surface of a carrier such as a substrate or a bead, immobilizing probe molecules on the metal particles, and modifying the probe molecules with fluorescent molecules. Prior to a reaction between the probe molecules and target molecules, a linear distance between the metal particle and the fluorescent molecule is set equal to or below 5 nm so as to transfer excitation energy from the fluorescent molecule to the metal particle before the target molecules react with the probe molecules. As a result, the fluorescence is quenched efficiently. Meanwhile, the distance between the metal particle and the fluorescent molecule is increased after the reaction so as to allow the fluorescent molecule to emit fluorescence.

Moreover, a fluorescence-enhancement effect is achieved by use of the probe molecule having a distance, along the probe molecule, between the fluorescent molecule modified on the probe molecules and the metal particle in a range from about 5 nm to about 100 nm. The "distance" stated herein means the distance between the center of mass of the metal particle and the center of mass of the fluorescent molecule. In a case where the probe molecule is a nucleic acid, the nucleic acid immobilized on the metal particle preferably has a hairpin structure.

As for the metal in the metal particle, it is possible to use noble metal, noble metal alloy or a lamination of noble metal substances. The diameter of the metal particle is set preferably in a range from 0.6 nm to 1 $\mu$m inclusive. In order to achieve measurement at a high ratio (a contrast) of fluorescent intensity before and after the reaction with the biomolecule, it is suitable to set the diameter of the metal particle in a range from 5 nm to 50 nm inclusive.

Meanwhile, in the present invention, the biomolecule is detected at ultrahigh sensitivity by utilizing a fluorescence-enhancement effect attributable to the metal particle. To achieve a high fluorescence-enhancement effect, when a localized plasmon resonance wavelength of the metal particle is defined as $\lambda$, an excitation wavelength $\lambda_E$ (nm) of the fluorescent molecule is set in a range of

$$\lambda - 100 < \lambda_E < \lambda + 100.$$

To achieve the high fluorescence-enhancement effect, the diameter of the metal particle is set preferably in a range from 10 nm to 500 nm inclusive.

Adsorption of the biomolecule causes a background noise from a nonspecifically adsorbed probe molecule. The adsorption of the biomolecule is suppressed by immobilizing a blocking agent molecule for suppressing adsorption of the probe molecule and the biomolecule on the surface of the carrier. Moreover, nonspecific adsorption of the biomolecule to be reacted is suppressed by immobilizing the blocking agent molecule on a surface of the metal particle.

According to the present invention, it is possible to detect a biomolecule for detection without labeling the biomolecule. In addition, by the highly sensitive detection utilizing the fluorescence-enhancement, it is also possible to detect the biomolecule for detection without amplification thereof in advance. By omitting preprocesses such as the labeling modification or the amplification treatment as described above, it is possible not only to improve quantitative reliability and repeatability of the biomolecule detection significantly but also to simplify the detection process.

BRIEF DESCRIPTION OF THE DRAWINGS

These and other features, objects and advantages of the present invention will become more apparent from the following description when taken in conjunction with the accompanying drawings wherein:

Fig. 1 is an explanatory drawing showing a basic configuration of a surface of a biosensor element according to the present invention;
Figs. 2A to 2E are conceptual drawings for explaining steps to manufacture the biosensor element according to the present invention;
Figs. 3A to 3E are conceptual drawings for explaining a concrete example of the steps to manufacture the biosensor element according to the present invention;
Fig. 4 is an explanatory graph for explaining a correlation between pH of an immobilizing solution when immobilizing polyethylene glycol functioning as a blocking agent molecule and a DNA adsorption amount after blocking;
Fig. 5 is an explanatory drawing for explaining a hairpin structure of a probe molecule;
Figs 6A and 6B are explanatory drawings showing states before and after a reaction between the probe molecules and biomolecules;
Figs. 7A and 7B are explanatory drawings showing fluorescence images of spots and average values of fluorescent intensity in the spot before and after hybridization;
Fig. 8 is a schematic drawing for explaining a bead array of the present invention;
Figs. 9A and 9B are explanatory graphs for explaining variation in the fluorescent intensity of the beads before and after hybridization;
Fig. 10 is an explanatory graph showing a relationship between diameters of gold nanoparticles and fluorescent intensity per fluorescent molecule (Cy3) when using 18-mer probe DNA;

Fig. 11 is an explanatory graph showing a relationship between the diameters of the gold nanoparticles and the fluorescent intensity per fluorescent molecule (Cy3) when using 50-mer probe DNA;

Fig. 12 is an explanatory graph showing a relationship between the diameters of the gold nanoparticles and the fluorescent intensity per fluorescent molecule (Cy5) when using the 50-mer probe DNA;

Fig, 13 is an explanatory graph for explaining a relationship between the diameters of the gold nanoparticles and contrasts;

Fig. 14 is an explanatory graph showing a relationship between target DNA concentrations and the contrasts;

Fig. 15 is an explanatory drawing showing a relationship between fluorescence-quenching field and fluorescence-enhancing field around a gold nanoparticle;

Figs. 16A and 16B are explanatory graphs showing a relationship between the diameters of the gold nanoparticles and fluorescent intensity coefficients;

Fig. 17 is an explanatory drawing showing an example of the surface of the biosensor element according to the present invention, which is the surface including the gold nanoparticles and the probe DNA immobilized thereon; and

Fig. 18 is an explanatory drawing showing a state after a reaction of fluorescence-labeled target DNA with the surface including the gold nanoparticles and the probe DNA immobilized thereon.

## DETAILED DESCRIPTION OF PREFERRED EMBODIMENTS

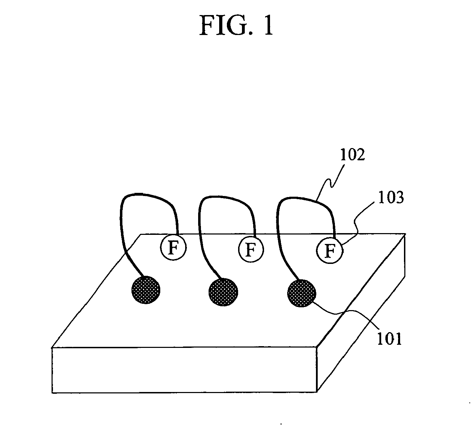

Fig. 1 is a view showing an example of a surface structure of a biosensor element according to the present invention. This biosensor element has the structure in which metal particles 101 are immobilized on a surface of a carrier, and in which probe molecules 102 modified with fluorescent molecules 103 are immobilized on surfaces of the metal particles. The probe molecules 102 are curved such that the fluorescent molecules 103 approach the metal particles 101.

First, a method of manufacturing the biosensor element will be described.

The method of manufacturing the biosensor element of the present embodiment includes the following steps 1 to 7:

1. Step for cleaning carrier;
2. Step for introducing active group onto carrier surface;
3. Step for immobilizing metal particles;
4. Step for blocking carrier surface;
5. Step for immobilizing probe molecules on metal particles;
6. Step for blocking on metal particles; and
7. Step for controlling structures of probe molecules.

Now, the respective steps will be described below.

### 1. Step for cleaning carrier

A carrier corresponding to a purpose is prepared, and subjected to cleaning. To be more precise, the carrier is cleaned with an alkaline aqueous solution such as a NaOH aqueous solution, and then cleaned with an acidic aqueous solution such as an HCl aqueous solution. The carrier is rinsed with purified water, and thereafter is subjected to drying under reduced pressure. Alternatively, organic contamination is rinsed off with a solution formed by blending sulfuric acid and hydrogen peroxide at an approximate proportion of 4 to 1.

As for the carrier, it is possible to use a glass substrate (a glass slide), a quartz substrate, a plastic substrate or the like. It is also possible to apply a metal-coated substrate, for example. It is preferable that the material of the carrier have a silanol group on a surface thereof. The carrier does not have to be of a flat form. For example, it is also possible to use the carrier of a bead form, a fiber form, a powder form or the like. In a case of the bead form, it is possible to use a carrier of a plastic bead such as polystyrene, a metal-coated bead, a magnetic bead or the like.

### 2. Step for introducing active group onto carrier surface

A silane coupling agent containing a reaction-active group is brought into a reaction with the surface of the cleaned carrier, thereby immobilizing the active group on the surface of the carrier.

As for the silane coupling agent, it is possible to use 3-aminopropyltrimethoxysilane, 3-aminopropyltriethoxysilane, N-(2-aminoethyl)-3-aminopropyltrimethoxysilane or (aminoethyl-aminomethyl) phenethyltrimethoxysilane, for example, in a case of immobilizing an amino group on the surface of the substrate. Meanwhile, it is possible to use 3-mercaptopropyltrimethoxysilane in a case of immobilizing a thiol group on the surface of the substrate.

As for a solvent, it is possible to use ethanol, methanol, toluene, benzene or water, for example. A reaction temperature is usually set in a range from 20°C to 85°C.

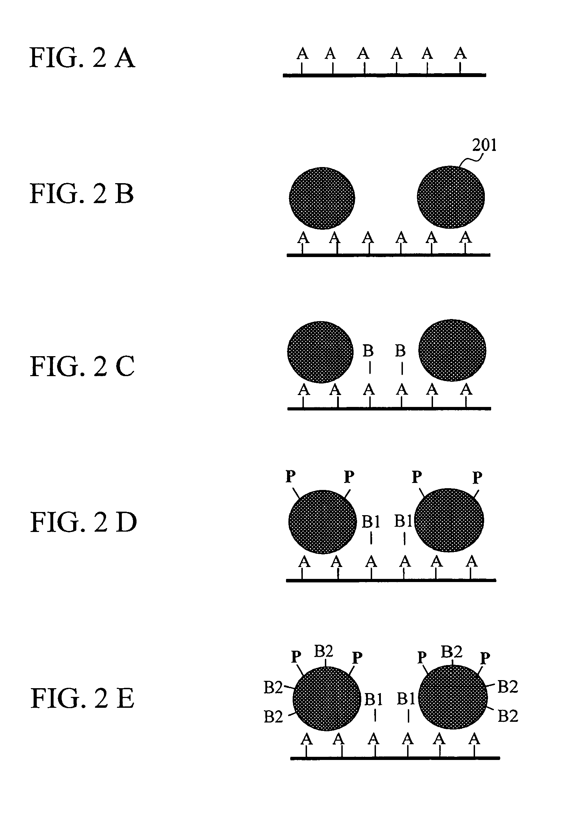

Fig. 2A is a schematic drawing showing an aspect of the surface of the carrier after introducing the active group while using the glass substrate as the carrier. In the drawing, reference code A denotes an immobilized molecule on a first layer. An amino group exists at a terminal of each molecule A, for example.

3. Step for immobilizing metalparticles

The metal particles are immobilized on the surface of the carrier by means of an interaction between the active groups and the metal particles on the surface of the carrier. Fig. 2B is a schematic drawing showing an aspect of the surface of the carrier on which metal particles 201 are immobilized.

As for the material of the metal particles, it is possible to use any of noble metal having fluorescence-quenching and fluorescence-enhancement effects including any of gold, silver, platinum, palladium, rhodium, iridium, ruthenium, and osmium, or alloy thereof. Alternatively, it is possible to use a metal particle which is made by these noble metals and which is coated with a noble metal with another kind such as a gold particle coated with silver. The diameter of the metal particles is set equal to or above 0.6 nm so as to permit continued existence of the metal particles and equal to or below 1 $\mu$m so as to achieve a fluorescence-enhancement effect attributable to localized plasmon resonance.

Water, ethanol or toluene can be used as a reaction solvent for immobilizing the metal particles. A protective agent is used to avoid aggregation of the metal particles in the solution. As for the protective agent, it is possible to use citric acid, mercaptosuccinic acid, polyvinylpyrrolidone, polyacrylic acid, tetramethylammonium, polyethyleneimine, 1-decanethiol, 1-octanethiol or decylamine, for example.

The concentration of the metal particles is usually set equal to or below 30 wt%. The reaction temperature is usually set in the range from 20°C to 85°C. Meanwhile, reaction time is set in a range from 0.5 hours to 50 hours. It is possible to control immobilization density of the metal particles by changing these conditions of reaction. For example, the immobilization reaction of the metal particles is a primary reaction for the concentration of the metal particles in an immobilizing solution, which is equivalent to a Langmuir-type reaction. Accordingly, it is possible to achieve desirable immobilization density by changing the concentration of the metal particles in the immobilizing solution or changing the reaction time. The immobilization density has to be equal to or above 1 particle/$\mu$m$^2$ equivalent to the metal particle density that allows detection of the fluorescent intensity. In the meantime, the immobilization density is set preferably equal to or below $10^6$ particles/$\mu$m$^2$ equivalent to the metal particle density for achieving single-layer saturated immobilization.

It is also possible to form and immobilize the metal particles on the surface of the carrier by use of a combination of techniques of vapor deposition, sputtering, chemical vapor deposition (CVD), annealing, and the like. In this case, the size and density of the metal particles to be immobilized on the carrier are determined by the conditions of vapor deposition, sputtering, CVD, and annealing. For example, it is possible to control the size and the density by changing a vapor deposition temperature, a duration of vapor deposition, a pressure during vapor deposition, an amount of vapor deposition, a duration of sputtering, a gas source used for CVD, a pressure and a temperature when performing CVD, a duration of CVD, an annealing temperature, a duration of annealing, and so forth. In order to improve adhesion between the carrier and the metal particles, it is also possible to interpose a spacer such as a chromium thin film between the carrier and the metal particles.

The diameter of the metal particle stated herein means not only the diameter of a spherical particle but also the diameter in a minor axis direction of a spheroidal or columnar particle.

4. Step for blocking carrier surface

Among the active groups formed on the surface of the carrier, an active group that remains on the surface without immobilizing the metal particle thereon is apt to adsorb a biomolecule in a later process. For this reason, such a remaining active group needs to be blocked. Fig. 2C is a schematic drawing showing an aspect of the surface of the carrier after a blocking process, in which blocking molecules are indicated with reference code B1.

For example, a molecule containing a polyethylene glycol chain is used as the blocking molecule. As for the molecule containing the polyethylene glycol, it is possible to use carboxyl polyethylene glycol having an N-hydroxyl succinimide (NHS) activated ester at a terminal. As the binding form of the carboxyl group, it is possible to apply any of succinate, glutalate, carboxylmethyl, and carboxylpentyl. Alternatively, it is also possible to use aldehyde polyethylene glycol having an aldehyde group at a terminal. The polyethylene glycol molecules used herein typically have molecular weights equal to or below 10000.

It is also possible to suppress adsorption of the biomolecules by using bovine serum albumin (BSA) or phospholipid polymer as the other blocking molecule. However, in a case of using the aforementioned polyethylene glycol, it is possible to immobilize the blocking molecule on the surface of the substrate by covalent binding, and thereby to form a stable blocking layer. Moreover, the polyethylene glycol has a significant effect of suppressing adsorption of the biomolecules such as nucleic acids or proteins.

Now, an example will be described below in a case where the active group on the surface of the substrate is an amino group and where carboxylmethyl polyethylene glycol (molecular weight equal to 2000) containing the NHS activated ester is used as the blocking molecule.

In this case, the amino group and the NHS activated ester group are brought into a reaction. As disclosed in Bioconjugate Techniques, Elsevier Science, p. 140 (1996), the NHS activated ester group is easily hydrolyzed with an alkaline solution. On the other hand, in order to cause the reaction between the amino group and the NHS activated ester, it is essential to cause the reaction in the pH range where the amino group is not protonated, i.e., in the alkaline solution. For this reason, it has been important to find out an appropriate pH range suitable for a polyethylene glycol reaction solution.

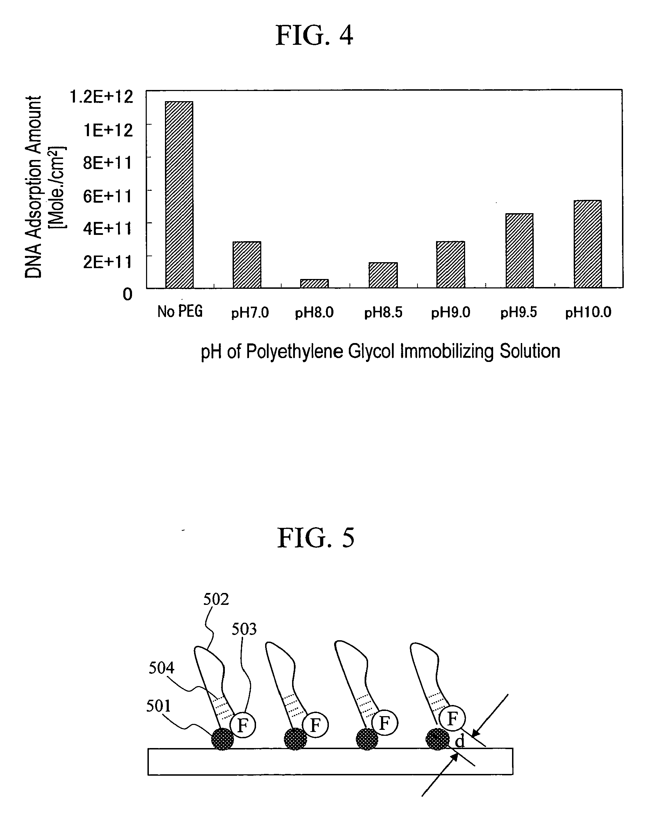

Fig. 4 shows a relation between the pH of the polyethylene glycol reaction solution and a nonspecific adsorption amount of the nucleic acid. As it is apparent from Fig. 4, the nonspecific adsorption amount is suppressed to 25% or below in a pH range of the reaction solution from 7.0 to 9.0, and the polyethylene glycol molecules exert the blocking effect if the reaction solution in this range is used for the blocking reaction.

Here, a triethanolamine solution, hydrochloric acid, a sodium carbonate solution or a sodium bicarbonate solution can be used as a pH adjuster for the polyethylene glycol solution. The reaction temperature is usually set in a range from 4°C to 35°C. The concentration of the polyethylene glycol reaction solution is usually set in a range from 1 mM to 100 mM, and the reaction time is set in range from 10 minutes to 120 minutes.

5. Step for immobilizing probe molecules on metal particles

The probe molecule having a group that can bind to the metal particles are brought onto the metal particles immobilized on the surface of the carrier to induce a reaction, thereby immobilizing the probe molecules on the metal particles. Fig. 2D is a schematic drawing showing an aspect of the surface of the carrier in which the probe molecules are indicated with reference code P.

Now, described will be a case of using a gold nanoparticle as the metal particle while using probe DNA having a thiol group at the 5' terminal thereof as a probe molecule. The 3' terminal of this probe DNA is modified with a fluorescent molecule. In general, when a linear distance between the metal particle and the fluorescent molecule is set equal to or below 5 nm, a speed of transfer of excitation energy from the fluorescent molecule to the metal particle is considerably increased. Thus, it is possible to quench the fluorescence. The transfer speed of the excitation energy from the fluorescent molecule to the metal particle is in inverse proportion to the sixth power of the distance between the metal particle and the fluorescent molecule. For this reason, a quenching effect becomes greater as the distance between these substances gets closer.

In a case of using the quenching probe DNA, it is possible to quench the fluorescence efficiently if an immobilized probe DNA 502 has a hairpin structure as illustrated in Fig. 5. Note that, the "energy transfer speed" is determined by a forester distance which is defined as a distance where the life time or the energy transfer ratio belonging to each fluorescent molecule becomes 50%. Therefore, the linear distance between a metal particle 501 and a fluorescent molecule 503 equal to or below 5 nm is in the range which the quenching effect has a large influence on. However, the quenching efficiency varies within that range depending on the types and sizes of the fluorescent molecules and the metal particles used therein.

Here, a "fluorescent material" means a substance that emits fluorescence when energy is applied thereto. The fluorescent material may be a cyanine dye such as Cy3 or Cy5, a rhodamine dye, a fluorescein dye or a material doped with erbium ions, for example. However, the fluorescent material will not be limited only to the foregoing substances. A single piece of the fluorescent material may be bonded to a single probe molecule. Otherwise, multiple pieces of the fluorescent material may be bonded to a single probe molecule. The fluorescent material may be bonded covalently to the probe molecule or by way of hydrogen bonding, coordinate bonding or ion bonding. Alternatively, the fluorescent material may be adsorbed to the probe molecule by way of physical adsorption.

Now, the immobilized probe DNA has a sequence in which 5 bases at both terminals are mutually complementary, such as:

TCCGC AAAAAAAAAAAAAAAAAAAA GCGGA

In this respect, the "complementary sequence" means a sequence which can form a stable pair by way of hydrogen bonding. Specifically, the complementary sequence is based on the complementary base pairs of A to T or T to A, and C to G or G to C.

In a case where both of the terminals include the mutually complementary sequences, these two terminals collectively form hydrogen bondings 504. Accordingly, it is easy to form the hairpin structure. The length of the complementary sequences from both of the terminals is set preferably equal to or below 8 bases. In a case where the length of the complementary sequences exceeds 8 bases, the hydrogen bondings at the terminals may be strong enough to form a stable structure. As a result, it may be difficult to cause this probe DNA to react with the biomolecule in the specimen.

Meanwhile, the quenching effect can be obtained even if the number of the complementary sequences at both of the terminals is equal to 0, because the fluorescence is quenched as the fluorescent molecule is adsorbed by the metal particle even in a case where the hairpin structure is not formed by the complementary bonding of the bases in the probe DNA. Of the above-mentioned sequence of the probe DNA, a sequence of a poly-A portion which is a consecutive A-base sequence can be changed depending on a sequence of detection target DNA.

For example, in a case where double-stranded DNA is formed as a result of the reaction between this probe DNA and the biomolecule for detection, the distance between the gold nanoparticle and the fluorescent molecule becomes equal to or longer than 5 nm. As described above, since the fluorescence-quenching effect by the metal particle is in inverse proportion to the sixth power of the distance between the metal particle and the fluorescent molecule, the light emission from the fluorescent molecule can be observed in a case where the distance is increased. A fluorescence-enhancement effect attributable to the metal particle is obtained in the range of the distance from about 5 nm to about 100 nm. In this respect, a relationship between the distance in the range from about 5 nm to about 100 nm and a proportion of the fluorescence-enhancement is determined by the type of the fluorescent molecule used therein as well as by the type and size of the metal particle. In every system, the fluorescence-enhancement is observed in the range from about 5 nm to about 100 nm. However, there is a certain distance in the range from about 5 nm and about 100 nm where the highest enhancement effect is obtained.

The fluorescence is enhanced in a case where the distance of between the metal particle and the fluorescent molecule modified on the probe molecule is set in the range from about 5 nm to about 100 nm when the biomolecule reacts with the probe molecule. When one of the terminal of the probe DNA is immobilized on the gold nanoparticle while the other terminal thereof is modified with the fluorescent molecule, it is preferable to set the length of the probe DNA in the range from about 5 nm to about 100 nm.

An almost neutral aqueous solution such as a phosphate buffer is applicable as a solution for dissolving the probe DNA. The probe DNA is dissolved in this solution. The concentration of the probe DNA at this time is usually set in a range from 0.5 $\mu$M to 100 $\mu$M.

In a case where the carrier is made of a flat glass substrate and the gold nanoparticle is immobilized thereon, a reaction solution dissolving the probe DNA may be spotted in desired positions on the substrate. At this time, it is possible to spot multiple types of the probe DNA onto the substrate. In a case where the carrier is made of a bead, the bead may be immersed in the reaction solution dissolving the probe DNA.

The reaction temperature is usually set in a range from 25°C to 40°C. Meanwhile, the reaction time is usually set in a range from 2 hours to 24 hours. In order to prevent the solution from drying during the reaction, the reaction should be taken place in an environment where a humidity is properly maintained.

Since the gold easily binds to the thiol group, the probe DNA having the thiol group at the terminal is immobilized solely on the metal particle. A portion where no gold nanoparticle is immobilized is covered with polyethylene glycol. Accordingly, the probe DNA is hardly adsorbed on that portion.

In this example, the metal particles are firstly immobilized on the substrate. Then the surface of the substrate where the metal particles are not immobilized is subject to blocking. Thereafter, the probe molecules are immobilized thereon. Instead, it is also possible to firstly immobilize the probe molecules on the metal particles, then to immobilize the metal particles on the surface, and then to block the surface of the substrate where the metal particles are not immobilized.

6. Step for blocking on metal particles

A portion on the surface of the metal particle where the probe DNA is not immobilized may adsorb the biomolecule for detection. For this reason, the remaining surface of the metal particle is subjected to blocking. Fig. 2E is a schematic drawing showing an aspect of the surface of the carrier after performing a blocking treatment on the surfaces of the metal particles in which a blocking agent is indicated with reference code B2.

Described will be a case of using the gold nanoparticles as the metal particles. It is possible to use 1-mercaptohexanol, 2-mercaptoethanol, or the like as a blocking agent that reacts easily with gold and hardly adsorbs the biomolecule. An aqueous solution of 1-mercaptohexanol or 2-mercaptethanol is reacted with the surface of the carrier so as to immobilize the blocking material.

The reaction temperature is usually set in a range from 4°C to 35°C. Meanwhile, the reaction time is usually set in a range from 0.5 hours to 10 hours. In this reaction, in a case where the concentration of the blocking agent in the aqueous solution is high, the blocking agent reacts with the gold nanoparticles, and thereby covers the gold nanoparticles. In this way, the blocking agent weakens the binding force between the carrier and the nanoparticle. As a result, the metal particles are dispersed on the surface of the carrier, and precipitate on the surface. A relationship between the concentration of the blocking agent and the presence of the surface dispersion is shown in Table 1. Based on Table 1, the concentration of the blocking agent reaction solution is set equal to or below 100 $\mu$M.

The step 5 and the step 6 are conducted separately. However, it is also possible to conduct the steps 5 and 6 at the same time. Specifically, when immobilizing the probe molecules on the metal particles, it is possible to carry out immobilization at the same time by use of a solution dissolving the probe molecules as well as the blocking agent for the metal particles.

Table 1

| Concentration of blocking agent reaction solution | Presence of surface dispersion of gold nanoparticles |
| --- | --- |
| 10 mM | Present |
| 1 mM | Present |
| 100 $\mu$M | Partially present |
| 10 $\mu$M | Partially present |
| 1 $\mu$M | Not present |

7. Step for controlling structures of probe molecules

The structures of the probe molecules immobilized on the metal particles are controlled such that the fluorescence is efficiently quenched by the metal particles. As previously described in the step 5, the fluorescent molecule placed in the vicinity of the metal causes excitation energy of the fluorescent molecule to transfer to free electrons in the metal, and thereby quenches the fluorescence. The quenching effect is high in a case where the distance between the metal particle and the fluorescent molecule is extremely small. In contrast, the quenching effect is substantially reduced in a case where the distance is increased. In order to render the distance between the fluorescent molecule and the metal particle as small as possible and thereby to quench the fluorescence efficiently, the hairpin structure as shown in Fig. 5 is actively formed.

Described will be a case of using the gold nanoparticles as the metal particles 501 while using the probe DNA as the probe molecules 502. When the surface of the substrate, on which a probe DNA is placed, is exposed to a solution having low basic strength, the negatively charged bases by way of phosphate bases in the DNA repel each other. Accordingly, the probe DNA is stretched, and is hardly formed into the hairpin structure. It is necessary to form the hairpin structure, therefore, in a solution having appropriate ionic strength.

As this solution, it is possible to use sodium carbonate, potassium carbonate, sodium phosphate, potassium phosphate, magnesium chloride, and the like. The ionic strength of the solution is usually set in a range from 50 mM to 2 M. The temperature is usually set in a range from 25°C to 45°C, and the reaction time is usually set in a range from 0.5 hours to 5 hours. Thereafter, the substrate is taken out of the solution and dried. Alternatively, it is possible to preserve the substrate while keeping contact with the above-described ionic solution.

Next, a detection method using the biosensor element manufactured in accordance with the above-described steps 1 to 7 will be described below.

8. Step for evaluating hybridization

Excitation light is irradiated on a surface of the biosensor element manufactured in accordance with the above-described steps 1 to 7 by use of a fluorescent scanner so as to detect light emission from the surface. If the distance between the fluorescent molecule modified at the terminal of the probe molecule and the metal particle is small enough, the fluorescence is quenched, and detected fluorescent intensity is extremely small.

Next, a biomolecule for detection and the above-described biosensor element are brought into a reaction. To be more precise, the surface of the biosensor element is allowed to contact the solution dissolving the biomolecule for detection and to continue the reaction until reaching the equilibrium. Described will be a case of using the probe DNA as the probe molecule while using the nucleic acid as the biomolecule for detection.

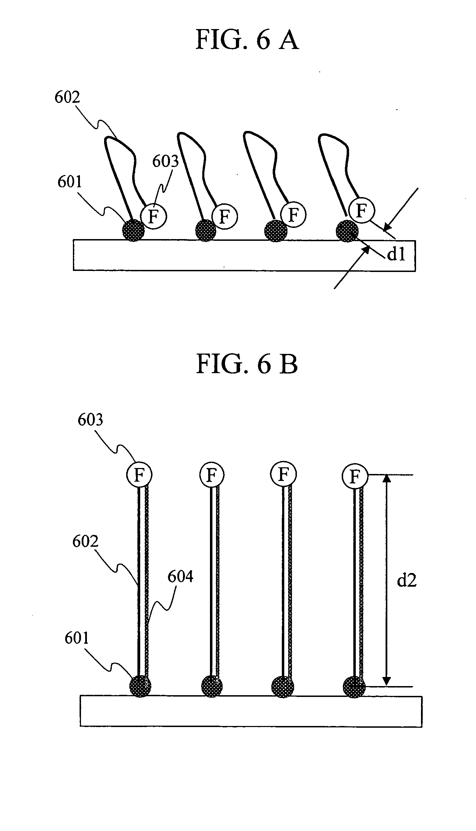

The nucleic acid for detection is dissolved in a surfactant-added standard saline citrate (SSC) solution, and this solution is allowed to contact the surface of the biosensor element. An amount of the nucleic acid in the solution is set in a range from 0.1 amol to 1 nmol. The reaction temperature is usually set in a range from 25°C to 60°C, and the reaction time is usually set in a range from 1 hour to 24 hours. In a case where the nucleic acid for detection is completely complementary to the sequence of the probe DNA, the nucleic acid reacts quickly to form double-stranded DNA that is linked by way of hydrogen bonding. The double-stranded DNA considerably loses flexibility as a polymer, and becomes like a rigid spring. After the reaction, a structural change occurs from a state illustrated in Fig. 6A to a state illustrated in Fig. 6B, thereby a distance between a metal particle 601 and a fluorescent molecule 603 immobilized on a probe DNA 602 is increased from a distance d1 to a distance d2. As a result, the fluorescent is emitted without being quenched. In the drawings, reference numeral 604 denotes a target molecule which is hybridized with the probe DNA 602. Moreover, the fluorescent intensity is enhanced by a localized plasmon resonance attributable to the metal particle. Now, the localized plasmon resonance and the fluorescence-enhancement will be described below.

When the light is irradiated on the metal particle, the free electrons in the metal particle are polarized and oscillated. Resonance between the oscillation of the free electrons in the metal particle and an oscillating magnetic field attributable to the incident light is called as the "localized plasmon resonance." When the localized plasmon resonance is generated, electric field intensity on the surface of the metal particle is increased by several digits as compared to the electric field intensity attributable to the incident light.

Next, two factors for the above-described fluorescence-enhancement will be described. A first factor for the fluorescence-enhancement is attributed to improvement in quantum efficiency of the fluorescent molecule. In a case where the metal particle is present in the vicinity of the fluorescent molecule, absorption transition occurs in the vicinity of the metal particle during the process of absorbing energy of the fluorescent molecule. The transition is attributable to an electric field enhancing effect owing to the localized plasmon resonance. In addition, the presence of the metal particle causes new light emission in a light emitting process.

Therefore, the quantum efficiency of the fluorescent molecule is enhanced by the absorption transition and the increase in the light emission. However, since the quantum efficiency never exceeds 1, the increase in the quantum efficiency attributable to the metal particle cannot be expected in a case of the fluorescent molecule that has the quantum efficiency equal to 1. Nevertheless, the fluorescent molecules used in the biosensor typically have the quantum efficiency in a range from about 0.04 to 0.3. Therefore, it is possible to expect the improvement in the quantum efficiency attributable to the metal particles when using these fluorescent molecules.

A second factor is an increase in light scattering intensity attributed to the metal particle. When polarizability of the metal particle is increased by the localized plasmon resonance and the electric field is enhanced in its vicinity, the light scattering intensity from the metal particle is also enhanced. This is attributed to the fact that the light scattering intensity is in proportion to the square of the polarizability of the metal particle. Along the increase in the light scattering intensity, incident energy for exciting the fluorescent molecule is also increased. Accordingly, fluorescence emission intensity is enhanced as well.

These fluorescence-enhancement effects are observed when the distance between the metal particle and the fluorescent molecule is in the range from 5 nm to 100 nm. In a case where the distance (d2) along the probe molecule between the end of the probe molecule immobilized on the metal particle and the fluorescent molecule modified on the probe molecule is in the range from 5 nm to 100 nm equivalent to the length where the fluorescence-enhancement effect is available, it is possible to utilize the fluorescence-enhancement effect after the probe molecule reacts with a specimen molecule. Accordingly, it is possible to detect the biomolecule in the specimen at ultrahigh sensitivity without labeling by highly efficient fluorescence-quenching and fluorescence-enhancement effects using the metal particles.

In accordance with this principle, in a case where the probe molecule 602 shown in Fig. 6A reacts with the biomolecule 604 and results in the structural change as shown in Fig. 6B, the fluorescence is enhanced because of the distance d2 between the fluorescent molecule 603 modified on the probe molecule and the metal particle 601. Although a fluorescence-enhancement ratio is a function of the distance d2, the fluorescence-enhancement ratio becomes a constant value because the distance d2 is defined as the length of the probe molecule and is always constant. It is, therefore, possible to find an amount of the biomolecules quantitatively by use of the enhanced fluorescent intensity.

In a case where the biosensor element formed by spot-immobilizing the multiple types of the probe DNA is used for measuring the intensity of fluorescent from the surface of the substrate after causing the biomolecules in the specimen to react with the probe DNA on the spots, fluorescence may be quenched on a certain spot whereas strong fluorescent intensity may be detected on another spot. From this result, it is apparent that the specimen does not contain the biomolecule related to the probe sequence on the spot where the fluorescence is quenched, and that the specimen contains the biomolecules related to the probe sequence on the spot where the strong fluorescent intensity is detected. Moreover, it is possible to quantitatively calculate the amount of the existing biomolecules by use of the measured fluorescent intensity.

Examples of the method of manufacturing the biosensor element of the present invention and the method of detecting a biomolecule using the element have been described. Although the embodiment has described the case of applying DNA as the biomolecule, it is also possible to apply other biomolecules such as RNA, proteins, PNA, sugar chains or composites thereof.

By using the biosensor element of the present invention, it is possible to detect the specimen molecules with excellent repeatability without amplifying and labeling them. Moreover, it is also possible to perform a quantitative analysis of an amount of gene expression, a highly selective analysis of SNPs, a highly selective analysis of proteins, or the like by use of the biosensor element of the present invention.

Next, the present invention will be described more in details with reference to examples. It is to be noted, however, that the following examples will not limit the scope of the present invention. The examples to be described below are based on the cases of applying the present invention to a flat-plate DNA microarray and to a bead array.

[Example 1]

(Step 1) Immobilization of metal particles on substrate

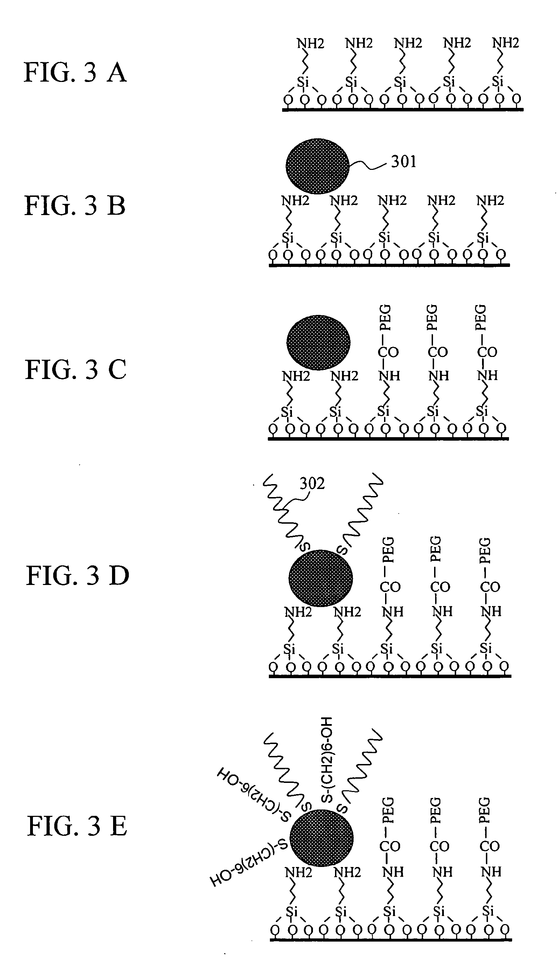

A glass slide made of borosilicate glass is prepared as a carrier. The substrate is cleaned with an NaOH aqueous solution, then cleaned with an HCl aqueous solution, then rinsed with purified water. Thereafter, it is subjected to drying under reduced pressure. As shown in Fig. 3A, 3-aminopropyltrimethoxysilane functioning as a silane coupling agent is allow to react with the cleaned surface of the substrate, thereby aminating the surface of the substrate. Note that, methanol is used as a solvent, and the concentration of the silane coupling agent is set equal to 3% (volume/volume). Meanwhile, the reaction temperature is set to room temperature, and the reaction time is set equal to 5 minutes.

Next, a citric acid solution containing gold nanoparticles in a diameter of 15 nm is brought onto the aminated substrate to effect a reaction. Note that, the concentration of the gold nanoparticles is set equal to 0.007% (weight/volume). Meanwhile, the reaction temperature is set to room temperature, and the reaction time is set equal to 20 hours. In this way, the substrate on which gold nanoparticles 301 are dispersed and immobilized was obtained as shown in Fig. 3B. The density of the gold nanoparticles at this time is set approximately equal to $1 \times 10^{11}$ pieces/cm$^2$.

(Step 2) Immobilization of blocking agent

Triethyl alcohol (TEA) in a concentration of 100 mM is adjusted to pH 8.0 by use of an HCl solution, and polyethylene glycol chains at a molecular weight of 2000 having succineimide-activated ester at terminals are dissolved in the solution. The multiple substrates on which the gold nanoparticles are immobilized as described above are immersed in the solution immediately after dissolving the polyethylene glycol chains. The reaction temperature is set equal to 25°C, and the reaction time is set equal to 1 hour. After the reaction, the substrates are cleaned with purified water, and subjected to drying under reduced pressure.

In this way, the substrate shown in Fig. 3C was obtained. After the polyethylene glycol was immobilized on the substrate at pH 8.0, the adsorption amount of 1 μM DNA was equal to or below $5 \times 10^{10}$ molecules/cm$^2$. Hence it was possible to reduce the adsorption amount approximately equal to or below 1/20 as compared to an adsorption amount on a substrate on which no polyethylene glycol was immobilized.

(Step 3) Immobilization of probe molecules

5 μM probe DNA having a base sequence of 30 to 60 pieces long and provided with a thiol group at the 5' terminal as well as Cy3 functioning as the fluorescent molecule at the 3' terminal was dissolved in a weak acidic phosphate buffer adjusted to pH 6.7 by mixing 50 mM of K$_2$HPO$_4$ with 50 mM of KH$_2$PO$_4$. The probe DNA dissolving solution was spotted at every probe DNA sequence onto the substrate which was subjected to blocking as described above. In this way, the substrate immobilizing multiple types of the probe DNA 302 thereon was obtained as shown in Fig. 3D. One of the probe DNA sequences was designed as: TCCGC AAAAA AAAAA AAAAA AAAAA GCGGA. Meanwhile, the sequence of a poly-A portion equivalent to a consecutive A-base sequence applied was a sequence corresponding to a sequence of detection target DNA. Specifically, the poly-A portion applied was any of a 17-mer sequence of AGAGATACATTGACCTT, a 21-mer sequence of CCCTTCTCACTGTTCTCTCAT or a 50-mer sequence of AGTCGAGCGGTAGCACAGAGAGCTTGCTCTCGGGTGACGAGCGGCGGACG, for example.

Note that, the reaction temperature is set equal to 25°C, and the reaction time is set equal to 4 hours. Moreover, in order to prevent the solution from drying during the reaction, the reaction was conducted in an environment where a humidity was properly maintained. After the reaction, the substrate was cleaned with purified water.

(Step 4) Immobilization of blocking agent on gold nanoparticles

A mercaptohexanol aqueous solution in a concentration of 1 μM was prepared, and the substrate immobilizing the probe DNA was immersed in this aqueous solution. The reaction temperature is set equal to 25°C, and the reaction time is set equal to 1 hour. After the reaction, the substrate was cleaned with purified water, and then subjected to drying under reduced pressure in a desiccator. In this way, the substrate shown in Fig. 3E was obtained.

(Step 5) Control of probe DNA structures

The surface of the substrate after blocking the surfaces of the gold nanoparticles as described above was immersed in a 2×SSC solution having basic strength of 0.3 M. The immersing temperature is set equal to 25°C, and the immersing time is set equal to 2 hours. Thereafter, the substrate was taken out of the solution and subjected to drying under reduced pressure. In the meantime, the 2×SSC solution was spotted on the same type of the substrate. By covering with a glass cover, the solution was allowed to contact the entire surface of the substrate. The substrate was preserved at 25°C.

(Step 6) Fluorescence measurement before reaction

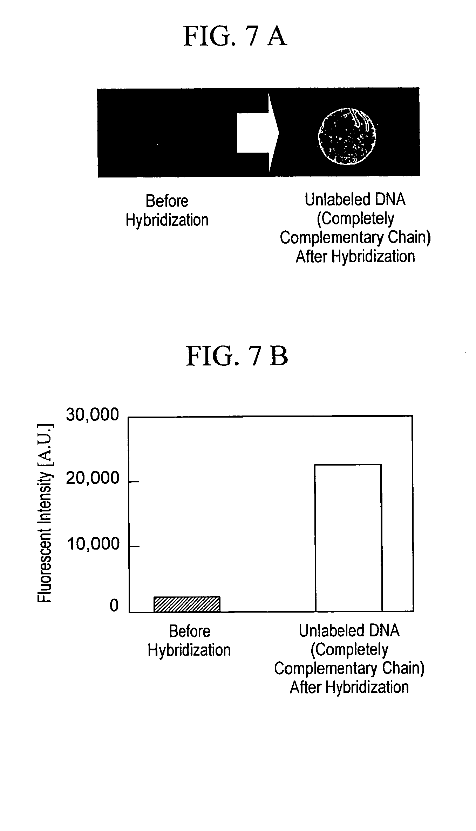

The fluorescent intensity from the probe DNA immobilized on the substrate was measured with the fluorescent scanner used in the step 5. A laser beam having a wavelength of 530 nm was used for scanning the surface of the substrate to excite the fluorescent dye Cy3, and strength of obtained fluorescence was measured. The results are shown in the sections titled "before hybridization" in Fig. 7A and Fig. 7B. Fig. 7A is a view showing a fluorescent image on one of the spots, and Fig. 7B is a graph showing average values of the fluorescent intensity in the spot. Note that, the fluorescent intensity shown in Figs. 7A and 7B represents the value measured after drying the substrate. In this respect, the fluorescent intensity was measured after drying the substrate. However, a similar result was obtained even when measuring the fluorescent intensity in the state of immersing the substrate in the solution as described in the step 5 without drying the substrate. In this case, the fluorescent intensity was measured while placing the solution on the substrate and covering the solution with a cover glass. In a case where using the cover glass, it is necessary to use the cover glass made of fused silica in order to suppress autofluorescence from the cover glass.
The surface capable of minimizing the probe DNA including nonspecifically adsorbed fluorescent molecules was constructed in accordance with the step 2. The probe DNA structures were controlled in accordance with the step 5. In this way, it was possible to suppress the fluorescence from the probe DNA, namely, the fluorescence subjected to background noises.

(Step 7) Hybridization

The substrate immobilizing the probe DNA thereon was subjected to hybridization with single-stranded target DNA having the completely complementary sequence to the probe DNA but not having a label thereon. A mixed solution of 5×SSC (standard saline citrate) and a 0.5% SDS (sodium dodecyl sulfate) solution was used as a hybridization solution, and a total amount fmol of the target DNA was hybridized at 42°C for 4 hours. Then, the substrate was cleaned with a 2×SSC, 0.1% SDS solution and with a 2×SSC solution and then subjected to drying under reduced pressure. The excitation light was made incident on the dried surface of the substrate with the fluorescent scanner, and the fluorescent intensity from the surface was measured.
In a case where the sequences of the target DNA and the probe DNA were completely complementary to each other, the fluorescent intensity was significantly increased after hybridization. The results are shown in the sections titled "unlabeled DNA after hybridization" in Fig. 7A and Fig. 7B. In this case, the target DNA has a sequence of CGTCCGCCGCTCGTCACCCGAGAGCAAGCTCTCTGTGCTACCGCTCGACT. Before hybridization, the fluorescence is quenched because the fluorescent molecule modified at the 3' terminal of the probe DNA is located close to the gold nanoparticle. However, it is apparent that the fluorescence is emitted without being quenched in a case where the rigid double-stranded DNA is formed by hybridization because the fluorescent molecule is separated from the gold nanoparticle. Moreover, it was possible to obtain high fluorescent intensity by the localized plasmon resonance effect attributable to the gold nanoparticle. In contrast, in a case where the sequence of the target DNA was not complementary to the sequence of the probe DNA, the fluorescence remained quenched after hybridization.

[Example 2]

In order to manufacture a bead array for a gene analysis, beads immobilizing the probe DNA were obtained in accordance with a method similar to the step 1 to the step 4 of the example 1 by use of beads instead of the substrate. Although the probe DNA was spotted in the step 3 of the example 1, the probe DNA was immobilized on the beads by immersing the beads in a solution dissolving the probe DNA. In this case, the probe DNA having a single type of sequence was immobilized on a single bead. Hence, multiple types of the beads were obtained by immobilizing multiple types of probe DNA on the multiple beads. The bead material used herein is made of borosilicate glass, and the diameter of each bead is approximately equal to 100 μm.

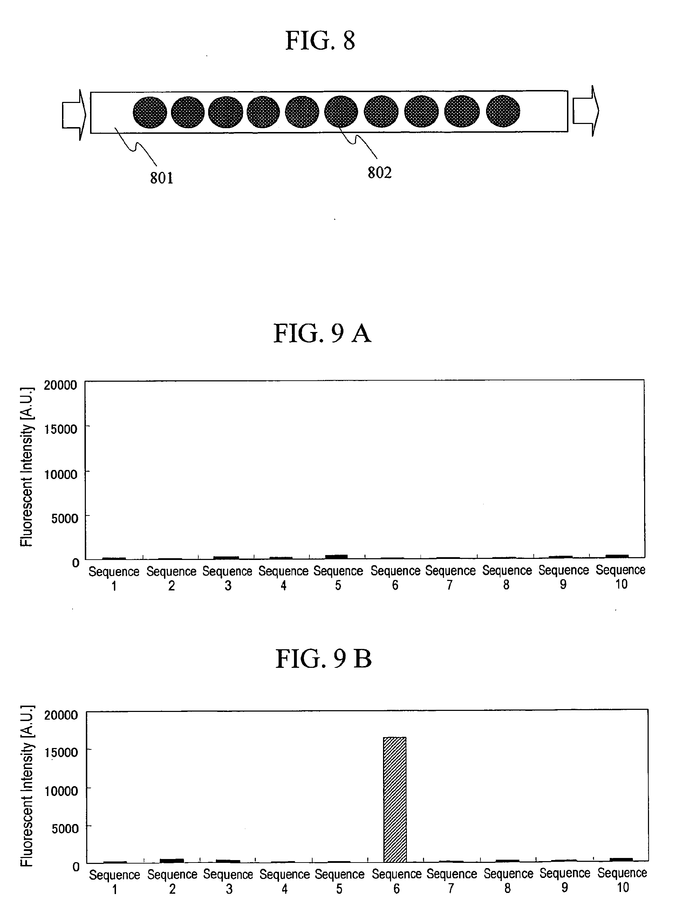

Fig. 8 is a schematic drawing showing a bead array for a gene analysis in a case of forming a single array by embedding ten types of beads 802, on which different types of probe DNA are immobilized in accordance with the above-mentioned manufacturing process, into a microchannel 801. The diameter of the microchannel 801 is approximately equal to 120 μm.

When the fluorescent intensity from surfaces of the beads before hybridization was measured in accordance with the method similar to the steps 5 and 6 of the example 1, it was confirmed that the fluorescence was quenched by the fluorescence-quenching effect attributable to the gold nanoparticles. The results are shown in Fig. 9A. The lateral axis in the graph of Fig. 9A or 9B indicates the types of the beads (the sequences of the probe DNA immobilized on the beads), and the longitudinal axis indicates the fluorescent intensity. It is obvious that very little fluorescence is observed from any of the beads.

Next, a sample fluid which contained target DNA having a completely complementary sequence to one of the multiple types of the probe DNA immobilized on the surfaces of the beads was poured in the microchannel 801 for the hybridization in accordance with the method of the step 7 of the example 1. Then, the fluorescent intensity and uniformity were examined. As a result, high fluorescent intensity was observed only out of the bead immobilizing the probe DNA which had the completely complementary sequence to the sequence of the target DNA as shown in Fig. 9B. In contrast, in terms of the rest of the probe DNA not having the complementary sequence to that of the target DNA, the fluorescence remained quenched after hybridization.

[Example 3]

To examine a difference in a gene detecting performance relative to the variation in the shapes of the metal particles, the gold nanoparticles and the blocking agent were immobilized on $SiO_2$ on the surface of the substrate in accordance with a method similar to the step 1 and the step 2 of the example 1. A substrate prepared by coating a gold thin film (about 50 nm) on glass and then coating $SiO_2$ on this gold thin film by sputtering in a thickness of 10 nm was used so that the substrate that can also measure surface plasmon resonance (SPR). While the step 1 of the example 1 applied only the gold nanoparticles having a diameter of 15 nm, the gold nanoparticles in various sizes were immobilized in this example, namely, those having diameters of 5 nm, 6 nm, 10 nm, 15 nm, 30 nm, 50 nm, and 80 nm. The concentration of the gold nanoparticle citric acid solution used therein is set to the gold content of 0.01% (weight/volume) in the case of the gold nanoparticle solution for the diameter of 5 nm, 6 nm or 10 nm, and the gold content of 0.007% (weight/volume) in the case of the gold nanoparticle solution for the diameter equal to or above 15 nm.

Providing that a distance between the centers of the mutually adjacent gold nanoparticles was denoted as "L" and that the diameter of each particle was denoted as "D," an immobilization interval between the gold nanoparticles L/D was approximately equal to 2 or greater in this case. There is a report that an adverse effect of an interaction between the particles is caused by electromagnetic fields around the particles that interfere with each other in a case where the interval L/D is close to 2 or below ("Interparticle Coupling Effects on Plasmon Resonances of Nanogold Particles," Nano Letters Vol. 3, No. 8, p. 1087-1090 (2003), "Optical Properties of Two Interacting Gold Nanoparticles," Optics Communications 220, p. 137-141 (2003), "Electrodynamics of noble metal nanoparticles and nanoparticle clusters," Journal of Cluster Science Vol. 10, No. 2 (1999)).

In this example, evaluation was conducted in a region where there was little effect of the interaction between the particles by setting the interval L/D approximately equal to 2 or greater. In other words, this example applied an immobilization surface with which it was possible to evaluate the effect of the diametric size of the gold nanoparticle on the gene detection performance directly.

Next, the probe DNA was immobilized on this substrate. To be more precise, the probe DNA was immobilized by use of the probe DNA dissolving solution described in the step 3. Although the DNA having the mutually complementary sequences at both terminals thereof was used as the probe DNA in the example 1, the probe DNA used in this example had any of the following 18-mer and 50-mer sequences not having the complementary sequences at both terminals thereof.

18-mer sequence: AGTCGAGCGGTAGCACAG

50-mer sequence:

AGTCGAGCGGTAGCACAGAGAGCTTGCTCTCGGGTGACGAGCGGCGGACG

Meanwhile, although the example 1 applied only Cy3 as the fluorescent molecules, this example applied two types of DNA modified with Cy3 and Cy5 at the 3' terminals. Each type of the probe DNA has a thiol group at the 5' terminal.

When immobilizing the probe DNA, measurement applying the surface plasmon resonance (SPR) was conducted in order to find amounts of immobilization of the probe DNA. Now, this SPR measurement method will be described below. The probe DNA immobilization is carried out by causing the reaction between the gold nanoparticles immobilized on the surface of the above-described substrate and the thiol groups at the terminals of the probe DNA. In this respect, when the light is made incident from the back side of the substrate, i.e. from the glass side, through an optical prism embedded in a SPR device, light reflectance is extremely reduced at a certain incident angle where the surface plasma oscillation on the surface of gold is induced. This angle is shifted depending on small variation in the mass (permittivity) on a surface of a sensor. Accordingly, it is possible to detect the amount of the probe DNA which is immobilized on the surface of the substrate by measuring an amount of the shift of this incident angle.

In this example, the substrates formed by immobilizing the gold nanoparticles in the sizes of 5 nm, 6 nm, 10 nm, 15 nm, 30 nm, 50 nm, and 80 nm thereon and immobilizing the blocking agent thereon were set in the SPR device. The solution containing the 18-mer probe DNA and the solution containing the 50-mer probe DNA which were adjusted to 10 $\mu$M were infused in the SPR device. Thereafter, the amounts of immobilization per unit area were calculated. Area occupancies of the probe DNA molecules on the surfaces of the gold nanoparticles, which were calculated by use of those amounts of immobilization, ranged from 1 $nm^2$ to 4 $nm^2$ in the case of the 18-mer probe DNA, and from 9 $nm^2$ to 14 $nm^2$ in the case of the 50-mer probe DNA.

Subsequently, the substrates were taken out of the SPR device, and the structures of the probe DNA immobilized on the substrates were controlled in accordance with a method similar to the step 5 of the example 1. Although the substrate was immersed in the 2$\times$SSC solution for 2 hours in the example 1, the substrates were immersed in the 5$\times$SSC solution for a period from 5 minutes to 2 hours in this example.

Next, the fluorescent intensity emitted from the probe DNA immobilized on the substrates was measured by use of the fluorescent scanner in accordance with a method similar to the step 6 of the example 1. Although the surface of the substrate was scanned by irradiating the laser beam having the wavelength of 530 nm in the example 1, a laser beam having a wavelength of 635 nm was used as the excitation light when applying Cy5 to the fluorescent molecules while a laser beam having a wavelength of 532 nm was used as the excitation light when applying Cy3 to the fluorescent molecules. Thereafter, the fluorescent intensity emitted from Cy3 or Cy5 was measured. In this respect, the fluorescent intensity detected in one pixel of 10 $\mu$m x 10 $\mu$m was measured. This fluorescent intensity measurement was carried out while immersing the surface of each substrate in the 5$\times$SSC solution.

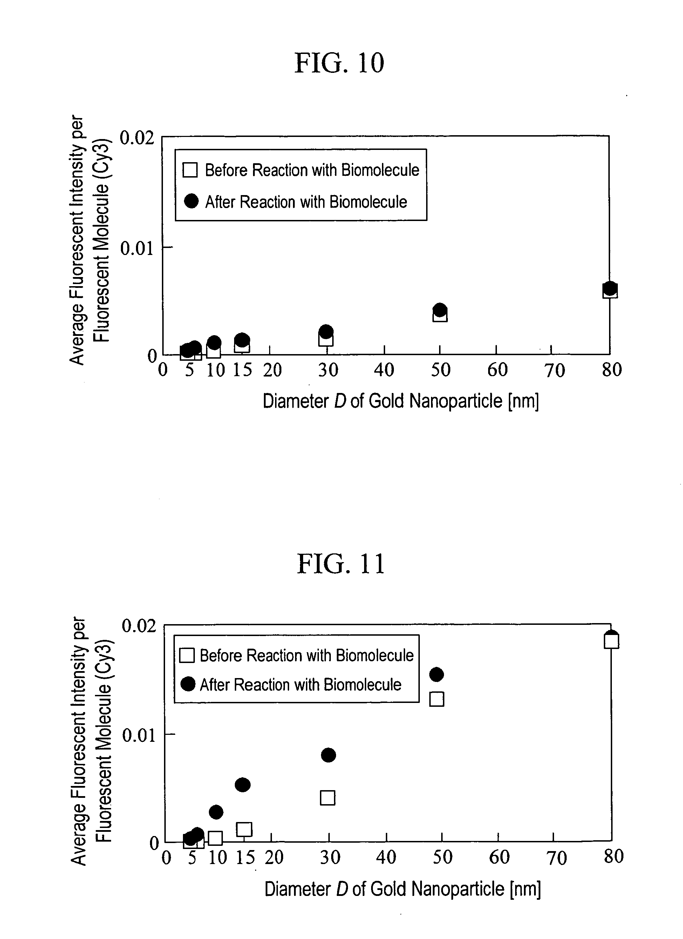

The fluorescent intensity per piece of the probe DNA, i.e. the fluorescent intensity per fluorescent molecule can be calculated by use of the number of the immobilized probe DNA molecules detected in one pixel of 10 $\mu$m $\times$ 10 $\mu$m obtained from a result of the above-mentioned SPR measurement and the fluorescent intensity obtained with the fluorescent scanner. Calculated values of the fluorescent intensity per fluorescent molecule are shown in Fig. 10 to Fig. 12.

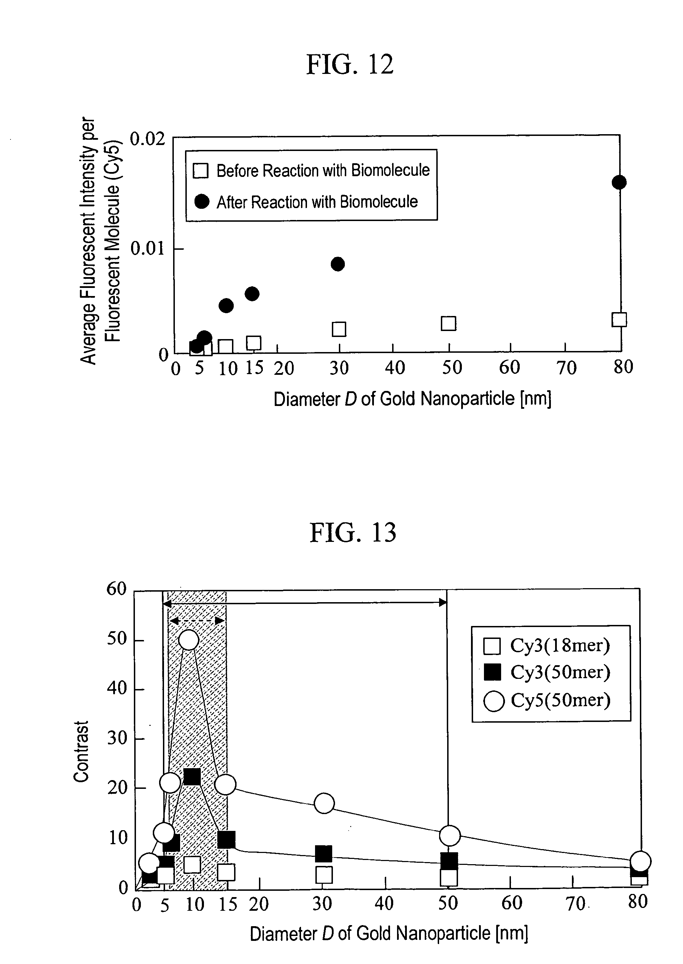

Fig. 10 is a graph showing a relationship between the diameters of the gold nanoparticles and the fluorescent intensity per fluorescent molecule (Cy3) when using the 18-mer probe DNA. Fig. 11 is a graph showing a relationship between the diameters of the gold nanoparticles and the fluorescent intensity per fluorescent molecule (Cy3) when using the 50-mer probe DNA. Fig. 12 is a graph showing a relationship between the diameters of the gold nanoparticles and the fluorescent intensity per fluorescent molecule (Cy5) when using the 50-mer probe DNA.

Next, single-stranded target DNA without fluorescence labeling was hybridized with the probe DNA immobilized on the above-described substrates in accordance with a method similar to the step 7 of the example 1. The sequences of the target DNA used in this example include completely complementary sequences and random sequences which do not have distinctive complementary sequences. The complementary sequences used therein are as follows.
18-mer sequence: CTGTGCTACCGCTCGACT
50-mer sequence:
CGTCCGCCGCTCGTCACCCGAGAGCAAGCTCTCTGTGCTACCGCTCGACT
Meanwhile, the random sequences used therein are as follows.
18-mer sequence: AAGTGAGCATCATTCACT
50-mer sequence:
TGAGTTTTTTAACCCGATGATTGTACTGCAACAAGTGAGCATCATTCACT
In the step 7 of the example 1, the cleaning and drying processes are executed after hybridization, and then the fluorescent intensity from the substrate is measured by use of the fluorescent scanner. In contrast, in this example, the substrates were cleaned with 5$\times$SCC solution after hybridization, and then the fluorescent intensity was measured while immersing the substrates in the 5$\times$SCC solution. A reason for conducting this operation is as follows. The fluorescent intensity varies depending on the permittivity of the solvent that surrounds the fluorescent molecule. Accordingly, in a case of comparing the fluorescent intensity between environments before and after hybridization more accurately, it is more appropriate to perform measurement while using the same solvent in both of the environments. In other words, it is more appropriate to perform measurement while immersing the substrates in the 5xSCC solution in both of the environments. In this case, it is possible to compare the fluorescent intensity between the environments before and after hybridization without considering an influence of the solvent surrounding the fluorescent molecule. Another reason is that it is possible to prevent denaturation of the DNA structures caused by the drying process. In this example, the amount of the target DNA was changed from 1 fmol to 1 pmol.

Values of average fluorescent intensity per fluorescent molecule when causing the reaction of 1 pmol of the target DNA are shown in Fig. 10 to Fig. 12. The following facts become obvious from these results. Before hybridization, the fluorescent intensity becomes small due to the fluorescence-quenching effect because the fluorescent molecule (Cy3 or Cy5) modified at the 3' terminal of the probe DNA is located close to the gold nanoparticle. Meanwhile, in a case where the rigid double-stranded DNA is formed by hybridization, the fluorescence is hardly quenched and the fluorescent intensity is therefore increased because the fluorescent molecule and the gold nanoparticle are separated.

A result of calculated contrast C (C = I2/I1) representing a ratio between the fluorescent intensity (I1) before hybridization and the fluorescent intensity (I2) after hybridization is shown in Fig. 13. In this respect, in a case where background noises B are unignorably large, the contrast applies the ratio which is obtained after subtracting the background noises respectively from I1 and I2. However, in this measurement, the simple ratio between I1 and I2 is defined as the contrast because the background noises are small.

A relation between the size of the gold nanoparticle and the contrast will now be explained with reference to Fig. 13. In a case where the contrast is large, i.e. in a case where it is possible to utilize the fluorescence-quenching effect and the fluorescence-enhancement effect favorably, it is possible to detect the biomolecule for detection at high sensitivity without labeling. From Fig. 13, it is apparent that the contrast is high in a region of the gold nanoparticles in a range from 5 nm to 50 nm inclusive. Moreover, it is apparent that the biomolecule can be detected at even higher contrast when the diameter of the metal particle is in a range from 6 nm to 15 nm inclusive.

In this respect, a reason of the higher contrast in the aforementioned range will be described below. To obtain high contrast, it is necessary to quench the fluorescence efficiently before hybridization and to enhance the fluorescence efficiently after hybridization. The degree of fluorescence-enhancement is increased along with an increase in the size of the gold nanoparticle. Since the polarizability of the metal particle is in proportion to the third power of a particle diameter, the polarizability is reduced in a case where the particle size is small, and electromagnetic strength around the particle is also reduced in this case. In a case where the particle diameter is equal to or below 5 nm, it is hard to obtain the fluorescence-enhancement effect, and is therefore hard to obtain high fluorescent intensity after the reaction with the biomolecule. In contrast, in a case where the particle diameter is equal to or above 50 nm, a strong fluorescence-enhancing field is generated around the particle. Accordingly, it is hard to cause fluorescence quenching, and high fluorescent intensity is obtained even before the reaction with the biomolecule. Thus, the ratio of the fluorescent intensity (the contrast) before and after the reaction with the biomolecule becomes small.

From this point of view, it is appropriate to set the particle diameter of the metal particle in the range from 5 nm to 50 nm inclusive in order to perform the highly sensitive measurement of the biomolecule at high contrast. To obtain even higher contrast, it is appropriate to set the particle diameter of the metal particle in the range from 6 nm to 15 nm inclusive.

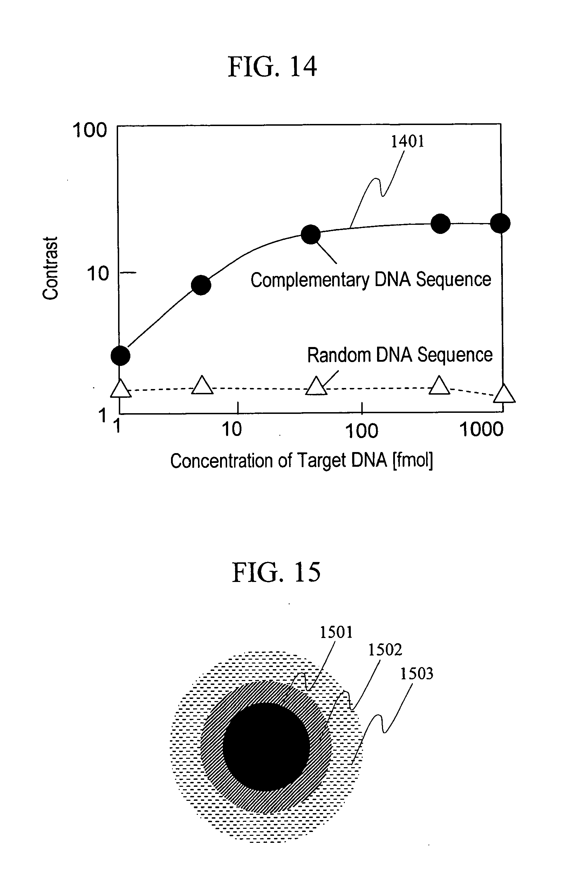

Next, a relation between the length of the probe DNA and the contrast will be explained. When comparing the contrast of the 50-mer probe DNA and the contrast of the 18-mer probe DNA, it is apparent that the contrast of the 50-mer probe DNA is the higher. A reason of this aspect will be described below. As shown in Fig. 15, a fluorescence-quenching field 1502 for quenching fluorescence by way of energy transfer to a nanoparticle from the fluorescent material exists in the vicinity of a metal particle 1501. Meanwhile, along with an increase in the electromagnetic field strength around the nanoparticle, a fluorescence-enhancing field 1503 also emerges in the vicinity of the gold nanoparticle as shown in Fig. 15. In this respect, a reason why the fluorescence-quenching field exists only in proximity to the gold nanoparticle is as follows. Specifically, the energy transfer speed that causes fluorescence quenching is in inverse proportion to the sixth power of the distance from the nanoparticle, and the energy transfer speed is drastically reduced along with the increase in the distance.

In a case where the probe DNA is 18-mer long, a chain length in a case of generating double-stranded chain is approximately equal to 6 nm. If the length is merely as long as 6 nm, the fluorescent molecule is located within the fluorescence-quenching field in proximity to the gold nanoparticle even after hybridization. Accordingly, the fluorescent molecule is not able to fully transit to the fluorescence-enhancing field where the stronger fluorescent intensity is obtainable. In contrast, in a case of the 50-mer probe DNA, a chain length of the generated double-stranded chain is approximately equal to 17 nm. Accordingly, the fluorescent molecule can transit to the fluorescence-enhancing field by forming the rigid double-stranded chain, and the contrast is enhanced as a consequence.

Fig. 14 shows a relationship between the contrast and the concentration of the 50-mer target DNA in the case of using the gold nanoparticles having the diameter of 15 nm that can obtain relatively high contrast. Apparently, it is possible to detect the target DNA selectively because only the DNA having the completely complementary sequence can achieve high contrast. Moreover, since the relationship between the contrast and the concentration of the target DNA succeeds in fitting accurately by use of a Langmuir model, it is possible to calculate the amount of the reacted biomolecules quantitatively from the magnitude of the contrast.

In this example, the substrate prepared by coating the gold thin film (about 50 nm) on the glass and then coating $SiO_2$ on this gold thin film by sputtering in a thickness of 10 nm was used as the substrate in order to perform the SPR measurement. However, it is also possible to obtain similar results on a substrate prepared by forming a $SiO_2$ thin film on a silicon substrate, on fused silica or on glass.

[Example 4]

In this example, the degree of the fluorescence enhancement, in a case of using the gold nanoparticles, was examined in order to detect genes at high sensitivity. To be more precise, a relationship between the degree of the fluorescence enhancement and the diameter of the gold nanoparticle was examined by quantitatively obtaining the degree of the fluorescence enhancement. An array used in this example was fabricated in accordance with a method similar to the method described in the example 3. Specifically, a substrate was prepared by coating $SiO_2$ on a gold thin film by sputtering in a thickness of 10nm and then various particle diameters of gold nanoparticles and probe DNA attaching fluorescent molecules (Cy3 or Cy5) were immobilized on the substrate to fabricate the array. This example applied the probe DNA having the following sequence:

50-mer sequence:

AGTCGAGCGGTAGCACAGAGAGCTTGCTCTCGGGTGACGAGCGGCGGACG

Meanwhile, in order to quantitatively evaluate the fluorescence-enhancement effect, another array was fabricated by immobilizing the probe DNA attaching the fluorescent molecules without using the gold nanoparticles. A functional group was coated on the surface of the substrate in accordance with a method similar to the step 1 of the example 1. Although 3-aminopropyltrimethoxysilane was used in the example 1, 3-mercaptopropyltrimethoxysilane was used in this example. Meanwhile, toluene was used as a reaction solvent. Next, the probe DNA was immobilized in accordance with a method similar to the step 3 of the example 1. The probe DNA has the same sequence as the above-mentioned sequence of the probe DNA immobilized on the gold nanoparticles. In this case, the probe DNA was bonded covalently to the substrate by forming disulfide bonds. In this way, the substrate on which the probe DNA attaching the fluorescent molecules was immobilized through the gold nanoparticles as well as the substrate on which the probe DNA attaching the fluorescent molecules was similarly immobilized without the gold nanoparticles were fabricated.

Next, immobilized amounts of the probe DNA were calculated by use of the surface plasmon resonance (SPR) in a case where the substrate was provided with the gold nanoparticles and in a case where the substrate was provided without the gold nanoparticles. As described previously, in the case where the gold nanoparticles were provided, the substrate to be used was formed as follows. The gold particles had the diameters of 5 nm, 6 nm, 10 nm, 15 nm, 30 nm, 50 nm, 80 nm, 100 nm, 200 nm, 300 nm, and 500 nm, and they were immobilized on the surfaces of the substrates. Thereafter, the blocking agent was immobilized thereon. On the other hand, in the case where the gold nanoparticles were not provided, the substrate to be used was coated with the thiol groups by way of 3-mercaptopropyltrimethoxysilane. These substrates were set on the SPR device, and then the above-described 50-mer probe DNA solution adjusted to 10 μM was infused thereon in the SPR device to measure the immobilized amounts per unit area.

Subsequently, hybridization was carried out on the above-described substrate by use of the substrates immobilizing the probe DNA thereon in accordance with a method similar to the step 7 of the example 1. DNA used as the target DNA had the completely complementary sequence to the probe DNA but no label. The target DNA used in this example had the following sequence:

50-mer sequence:

CGTCCGCCGCTCGTCACCCGAGAGCAAGCTCTCTGTGCTACCGCTCGACT

In the step 7 of the example 1, the cleaning and drying processes were executed after hybridization, and the fluorescent intensity was measured thereafter. In contrast, in this example, after the substrate was cleaned, the fluorescent intensity was measured while the substrate was immersed in the 5×SCC solution. The average fluorescent intensity per fluorescent molecule was calculated from the immobilized amounts of the probe DNA attaching the fluorescent molecules which were obtained by the above-described SPR measurement and from the measured fluorescent intensity. Note that, in this example, the amount of the target DNA used for the reaction was set equal to 1 pmol.

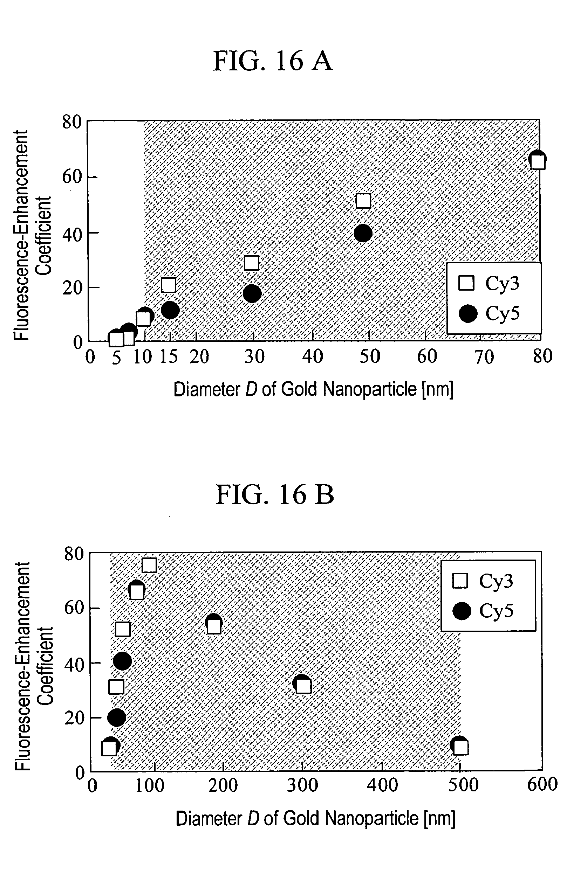

Fluorescent intensity (I3) per fluorescent molecule after hybridization using the substrate on which the gold nanoparticles were immobilized was compared with fluorescent intensity (14) per fluorescent molecule after hybridization using the substrate on which the gold nanoparticles were not immobilized. Here, the substrate on which the gold nanoparticles were immobilized had the higher fluorescent intensity. The results of obtaining fluorescence-enhancement coefficients E defined as E = I3/I4 are shown in Figs. 16A and 16B. In a case where the diameter of the gold nanoparticle was equal to or above 10 nm, it was possible to enhance the fluorescent intensity ten times or more. This is attributable to the fact that in a case where the polarizability of the metal particle is in proportion to the third power of the particle diameter, and where the particle is large, the polarizability is increased, and the electromagnetic field strength around the particle is also increased. It is, therefore, possible to obtain a high degree of fluorescence enhancement in a case where the diameter of the gold nanoparticle is equal to or above 10 nm.

On the other hand, in a case where the diameter of the gold nanoparticle becomes almost as large as the wavelength, the polarization hardly occurs in the gold nanoparticle and the electromagnetic field around the gold nanoparticle is also reduced along with reduction in the polarizability. Therefore, it is possible to obtain a high fluorescence-enhancement effect by using the gold nanoparticles having the particle diameters in a range from 10 nm to 500 nm inclusive.

Now, comparison between Cy3 and Cy5 will be considered. In a case where the diameter of the gold nanoparticle is smaller than 100 nm, the fluorescence-enhancement coefficient of Cy3 became greater than that of Cy5. A reason for this aspect will be described below. The gold nanoparticle exerts light absorption attributable to the localized plasmon resonance by the polarization thereof. In a case where the diameter of the gold nanoparticle is smaller than 100 nm, a wavelength for this absorption is equal to somewhere from 500 nm to 550 nm. In this wavelength band, the localized plasmon resonates with the light incident on the gold nanoparticle, thereby increasing light absorption or near-field scattering. In a case where the wavelength inducing the localized plasmon resonance is used as an excitation wavelength, the strength of the near-field light scattering is increased. Thus, it is conceivable that the fluorescent intensity is enhanced more. The excitation wavelength for Cy3 ranges from 500 nm to 550 nm which coincides with the localized plasmon resonance wavelength band. In contrast, the excitation wavelength for Cy5 ranges from 600 nm to 650 nm which deviates from the resonance wavelength band. It is, therefore, conceivable that the larger fluorescence enhancement is achieved with Cy3.

In this example, the substrate prepared by coating the gold thin film (about 50 nm) on the glass and then coating $SiO_2$ on this gold thin film by sputtering in a thickness of 10 nm was used as the substrate in order to perform the SPR measurement. However, it is also possible to obtain similar results on a substrate prepared by forming a $SiO_2$ thin film on a silicon substrate, on fused silica or on glass.

[Example 5]

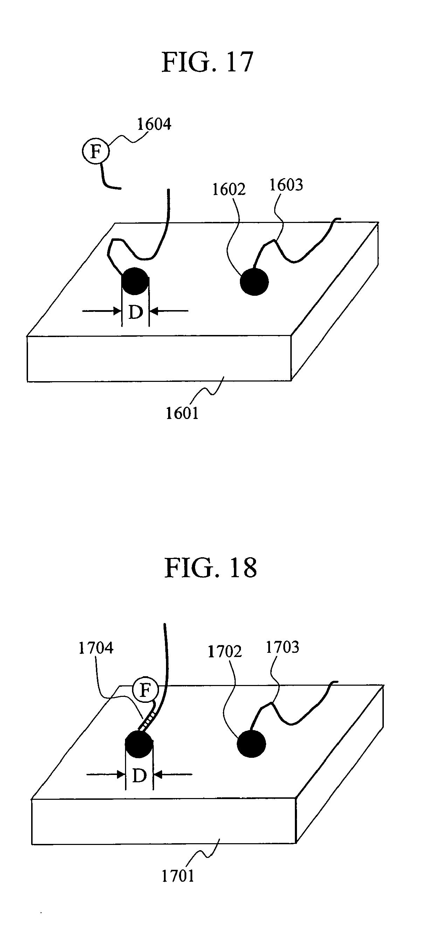

In this example, detection was attempted while utilizing the fluorescence enhancement by the gold nanoparticles for the purpose of highly sensitive detection of fluorescence-labeled genes. Fig. 17 is a schematic drawing showing a detection array used in this example. To manufacture the array for detection, the array surface was obtained in accordance with a method similar to the method described in the example 4 so that gold nanoparticles and probe DNA were immobilized thereon. Although the probe DNA attaching the fluorescent molecules was immobilized in the example 4, 50-mer probe DNA without fluorescent molecules was immobilized in this example.

Meanwhile, in order to quantitatively evaluate the fluorescence-enhancement effect, another substrate was manufactured by immobilizing the probe DNA without using the gold nanoparticles. As similar to the example 4, the probe DNA was immobilized after coating 3-mercaptopropyltrimethoxysilane. Although the probe DNA attaching the fluorescent molecules was immobilized in the example 4, the immobilized probe DNA did not attach the fluorescent molecules but had the same sequence as the above-described probe DNA immobilized on the gold nanoparticles in this example. In this way, the substrate on which the probe DNA was immobilized through the gold nanoparticles as well as the substrate on which the probe DNA was immobilized without the gold nanoparticles were fabricated.

Next, 1 pmol of single-stranded target DNA having a completely complementary sequence to the sequence of the probe DNA was subjected to hybridization by use of the substrates on which the above-described probe DNA was immobilized. The target DNA having no label was used in the example 4. Instead, in this example, the used target DNA was modified with the fluorescent molecules (Cy3 or Cy5) functioning as the label at the 3' terminals thereof. Fig. 18 is a schematic drawing showing the array after hybridization. After hybridization, the fluorescent intensity was measured in accordance with a method similar to the method described in the example 4.

In order to calculate the fluorescent intensity per reacted fluorescent molecule, a reacting amount of the hybridized target DNA was calculated at the time of the hybridization described above. Upon calculation of the reacting weight, the surface plasmon resonance (SPR) was used as similar to the method described in the example 3. The substrate fabricated for SPR measurement was set on the SPR device, then a solution containing the target DNA was infused thereon in the SPR device, and then an amount of the hybridized target DNA per unit area was calculated. The fluorescent intensity per hybridized fluorescent molecule was calculated from a measurement result of this SPR hybridized amount and the above-described measurement result of the fluorescent intensity.

The fluorescent intensity (I3) per fluorescent molecule after hybridization using the substrate on which the gold nanoparticles were immobilized was compared with the fluorescent intensity (I4) per fluorescent molecule after hybridization using the substrate on which the gold nanoparticles were not immobilized. Here, the substrate on which the gold nanoparticles were immobilized had the higher fluorescent intensity. As a result of obtaining the fluorescence-enhancing coefficients E defined as E= I3/I4, it was possible to enhance the fluorescent intensity ten times or more as similar to the example 4 in a case where the diameter of the gold nanoparticle was equal to or above 10 nm. Based on the relationship between the diameter of the metal particle and the polarizability described in the example 4, it was possible to obtain a high fluorescence-enhancement effect as similar to the example 4 by using the gold nanoparticles having the particle diameters in the range from 10 nm to 500 nm inclusive.

In this example, the substrate prepared by coating the gold thin film (about 50 nm) on the glass and then coating $SiO_2$ on this gold thin film by sputtering in a thickness of 10 nm was used as the substrate in order to perform the SPR measurement. However, it is also possible to obtain similar results on a substrate prepared by forming a $SiO_2$ thin film on a silicon substrate, on fused silica or on glass.

Moreover, the used target DNA had the completely complementary sequence to the sequence of the probe DNA in this example. However, it is also possible to obtain similar effects when a target molecule for detection applies a single-base sequence labeled with a fluorescent molecule such as dCTP-Cy3 or ddCTP-Cy3, a protein modified with a fluorescent molecule, a carbohydrate chain or glycoprotein modified with a fluorescent molecule, and so forth.

While we have shown and described several embodiments in accordance with out invention, it should be understood that disclosed embodiments are susceptible of changes and modifications without departing from the scope of the invention. Therefore, we do not intend to be bound by the details shown and described herein but intend to cover all such changes and modifications a fall within the ambit of the appended claims.

**Claims**

1. A biosensor element comprising:

   a metal particle (101) immobilized on a surface of a carrier;
   a probe molecule (102) immobilized on a surface of the metal particle (101); and
   a fluorescent molecule (103) modified on the probe molecule (102).

2. The element of claim 1, wherein a linear distance connecting the fluorescent molecule (103) modified on the probe molecule (102) to the metal particle (101) is equal to or below 5 nm.

3. The element of claim 1, wherein a distance between the fluorescent molecule (103) modified on the probe molecule (102) and the metal particle (101) along the probe molecule is in a range from 5 nm to 100 nm inclusive.

4. The element of claim 1, wherein

   one of terminals of the probe molecule (102) is immobilized on the metal particle (101), and
   the fluorescent molecule (103) is modified on the other end of the probe molecule (102).

5. The element of claim 1, wherein a particle diameter of the metal particle (101) is in a range from 0.6 nm to 1 $\mu$m inclusive, preferably in a range from 5 nm to 50 nm inclusive.

6. The element of claim 1, wherein the immobilization density of the metal particles (101) on the surface of the carrier is in a range from 1 particle/$\mu$m$^2$ to $10^6$ particles/$\mu$m$^2$ inclusive.

7. The element of claim 1, wherein

   a silane coupling agent molecule is immobilized on the surface of the carrier, and
   the metal particle (101) is immobilized on the silane coupling agent molecule.

8. The element of claim 1, wherein the surface of the carrier comprises:

   a portion on which the metal particle (101) is immobilized; and
   a portion on which a blocking agent molecule is immobilized.

9. The element of claim 1, wherein the surface of the metal particle (101) comprises:

   a portion on which the probe molecule (102) is immobilized; and
   a portion on which a blocking agent molecule is immobilized.

10. The element of claim 1, wherein

    the probe molecule (102) is a nucleic acid, and
    the nucleic acid forms a hairpin structure when the nucleic acid is immobilized on the surface of the metal particulate.

11. The element of claim 1, wherein

    the probe molecule (102) is a nucleic acid, and
    sequences of less than 8 bases located at both terminals of the nucleic acid sequence are mutually complementary to each other.

**12.** A biosensor element comprising:

a metal particle (101) immobilized on a surface of a carrier; and
a probe molecule (102) immobilized on a surface of the metal particle (101), the probe molecule being configured to bind to a fluorescence-labeled target molecule, wherein a diameter of the metal particle (101) is in a range from 10 nm to 500 nm inclusive.

**13.** The element of claim 1 or 12, wherein the metal particle (101) is made of any of metal belonging to noble metals, alloy of the metal belonging to the noble metals, and a laminate of the metal belonging to the noble metals.

**14.** A method of manufacturing a biosensor element having a probe molecule (102) immobilized on a surface of a carrier, the method comprising the steps of:

immobilizing a metal particle (101) on the surface of the carrier;
immobilizing a blocking agent molecule on the surface of the carrier; and
immobilizing the probe molecule (102) modified with a fluorescent molecule (103) on a surface of the metal particle (101).

**15.** The method of claim 14, wherein the step of immobilizing the metal particle (101) on the carrier comprises the steps of:

immobilizing a silane coupling agent molecule on the surface of the carrier; and
immobilizing the metal particle (101) by allowing a solution containing the metal particle to contact the surface of the carrier.

**16.** The element of claim 8 or the method of claim 14, wherein the blocking agent molecule contains a polyethylene glycol chain.

**17.** The method of claim 16, wherein an immobilization reaction solution dissolving the molecule containing the polyethylene glycol chain has a pH ranging from 7.0 to 9.0 inclusive in the step of immobilizing the molecule containing the polyethylene glycol chain.

**18.** The method of claim 14, further comprising the step of immobilizing a blocking agent molecule on the surface of the surface of the metal particle (101).

**19.** The method of claim 18, wherein the concentration of the blocking agent molecules in an immobilization reaction solution is equal to or below 100 $\mu$M.

**20.** A method of detecting a biomolecule by use of a biosensor element having a metal particle (101) immobilized on a surface of a carrier, a probe molecule (102) immobilized on a surface of the metal particle (101) and a fluorescent molecule (103) modified on the probe molecule, wherein the method comprising the steps of:

bringing the probe molecule (102) of the biosensor element and an unlabeled detection target biomolecule into a reaction;
irradiating excitation light on the biosensor element after the reaction; and
detecting fluorescence emitted from a region where the probe molecule (102) is immobilized.

**21.** The method of claim 20, wherein

the fluorescent molecule (103) modified on the probe molecule (102) approaches the metal particle (101) before the reaction to cause fluorescence quenching, and
the fluorescent molecule (103) recedes from the metal particle (101) after the reaction to emit fluorescence upon irradiation of the excitation light.

**22.** A method of detecting a biomolecule by use of a biosensor element having a metal particle (101) immobilized on a surface of a carrier, a probe molecule (102) immobilized on a surface of the metal particle and a fluorescent molecule (103) modified on the probe molecule, the method comprising the steps of:

irradiating excitation light on the biosensor element;

measuring first intensity of fluorescence emitted from a region of the biosensor element on which the probe molecule (102) is immobilized;

bringing the probe molecule (102) of the biosensor element and an unlabeled detection target biomolecule to a reaction;

irradiating the excitation light on the biosensor element after the reaction;

measuring second intensity of fluorescence emitted from the region of the biosensor element on which the probe molecule (102) is immobilized; and

calculating a contrast by dividing the second intensity of fluorescence by the first intensity of fluorescence.

23. The method of claim 22, wherein a diameter of the metal particle (101) is in a range from 10 nm to 500 nm inclusive.

24. A method of detecting a biomolecule by use of a biosensor element having a metal particle (101), with a diameter in a range from 10 nm to 500 nm, immobilized inclusive on the surface of a carrier, and a probe molecule (102) immobilized on a surface of the metal particle, the method comprising the steps of:

bringing the probe molecule (102) of the biosensor element and a fluorescence-labeled biomolecule to a reaction;

irradiating excitation light on the biosensor element; and

measuring intensity of fluorescence emitted from a region of the biosensor element on which the probe molecule (102) is immobilized.

25. The method of claim 20 or 24, wherein when a localized plasmon resonance wavelength of the metal particle (101) is defined as $\lambda$, an excitation wavelength $\lambda_E$ of the fluorescent label is in a range expressed as: $\lambda - 100$ nm$< \lambda_E < \lambda + 100$ nm.

FIG. 1

FIG. 2 A

FIG. 2 B

FIG. 2 C

FIG. 2 D

FIG. 2 E

FIG. 3 A

FIG. 3 B

301

FIG. 3 C

FIG. 3 D

302

FIG. 3 E

# FIG. 4

# FIG. 5

# FIG. 6 A

# FIG. 6 B

# FIG. 7 A

Before
Hybridization

Unlabeled DNA
(Completely
Complementary Chain)
After Hybridization

# FIG. 7 B

Before
Hybridization

Unlabeled DNA
(Completely
Complementary Chain)
After Hybridization

# FIG. 8

801          802

# FIG. 9 A

# FIG. 9 B

# FIG. 10

# FIG. 11

# FIG. 12

# FIG. 13

# FIG. 14

Contrast vs Concentration of Target DNA [fmol]

1401

Complementary DNA Sequence

Random DNA Sequence

# FIG. 15

1501
1502
1503

## FIG. 16 A

## FIG. 16 B

# FIG. 17

# FIG. 18

**European Patent Office**

**EUROPEAN SEARCH REPORT**

Application Number

EP 07 00 2203

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/059279 A (UNIV MARYLAND BIOTECHNOLOGY [US]; GEDDES CHRIS D [US]; LAKOWICZ JOSEPH) 15 July 2004 (2004-07-15) * pages 5-6 * * page 9, paragraph 1 * * claims 1-4,11,16,19,28,50 * * figure 1 * | 1-13 | INV. C12Q1/68 |
| D,X | MALICKA J ET AL: "DNA Hybridization Assays Using Metal-Enhanced Fluorescence" BIOCHEMICAL AND BIOPHYSICAL RESEARCH COMMUNICATIONS, ACADEMIC PRESS, SAN DIEGO, CA, US, vol. 306, 2003, pages 213-218, XP003013165 ISSN: 0006-291X * the whole document * | 12,13,24 | |
| X | ASLAN ET AL: "Microwave-Accelerated Metal-Enhanced Fluorescence (MAMEF) with silver colloids in 96-well plates: Application to ultra fast and sensitive immunoassays, High Throughput Screening and drug discovery" JOURNAL OF IMMUNOLOGICAL METHODS, ELSEVIER SCIENCE PUBLISHERS B.V.,AMSTERDAM, NL, vol. 312, no. 1-2, 30 May 2006 (2006-05-30), pages 137-147, XP005479376 ISSN: 0022-1759 * the whole document * | 12,13,24 | TECHNICAL FIELDS SEARCHED (IPC) C12Q |
| X | WO 02/064837 A (UNIV MARYLAND [US]) 22 August 2002 (2002-08-22) * paragraphs [0011] - [0019], [0062] - [0086], [0092] - [0101]; claims 1-27 * | 1-13,24 | |

-/--

The present search report has been drawn up for all claims

| Place of search | Date of completion of the search | Examiner |
|---|---|---|
| The Hague | 20 September 2007 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## EUROPEAN SEARCH REPORT

**European Patent Office**

**Application Number**

EP 07 00 2203

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 812 334 B1 (MIRKIN CHAD A [US] ET AL) 2 November 2004 (2004-11-02) * claims 1,9,10 * * figure 13B * * column 2, paragraph 3 * * column 8, paragraphs 4,5 * * column 19, paragraph 5 * * column 33, paragraph 3 * * example 6 * | 1-13, 20-23 | |
| X | US 2003/040000 A1 (CONNOLLY DENNIS M [US] ET AL) 27 February 2003 (2003-02-27) * figure 6 * * claim 27 * | 12,13 | |
| X | WO 2006/052548 A (UNIV MARYLAND BIOTECH INST [US]; GEDDES CHRIS D [US]; ASLAN KADIR [US]) 18 May 2006 (2006-05-18) | 1-13,24 | |
| Y | * pages 3-5, paragraph 5 * * page 7, paragraph 7 * * page 10, paragraph 3 * * page 13, paragraph 2 - page 19, paragraph 2 * * page 21, paragraph 4 * * claims 1,5-18 * | 14-19 | TECHNICAL FIELDS SEARCHED (IPC) |
| Y | WO 2005/098441 A (INTEL CORP [US]; KOO TAE-WOONG [US]; CHAN SELENA [US]) 20 October 2005 (2005-10-20) * paragraphs [0022], [0052], [0053] * | 14-19 | |
| Y | US 6 579 726 B1 (NATAN MICHAEL J [US] ET AL) 17 June 2003 (2003-06-17) * paragraph [0112] * | 14-19 | |

-/--

The present search report has been drawn up for all claims

| Place of search | Date of completion of the search | Examiner |
|---|---|---|
| The Hague | 20 September 2007 | Bruma, Anja |

**European Patent Office**

## EUROPEAN SEARCH REPORT

Application Number

EP 07 00 2203

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2005/019812 A (UNIV STRATHCLYDE [GB]; GRAHAM DUNCAN [GB]; SMITH WILLIAM EWEN [GB]; FR) 3 March 2005 (2005-03-03) * the whole document * | | |
| A | STROHSAHL ET AL: "Towards single-spot multianalyte molecular beacon biosensors" TALANTA, ELSEVIER, AMSTERDAM, NL, vol. 67, no. 3, 15 September 2005 (2005-09-15), pages 479-485, XP005047815 ISSN: 0039-9140 * the whole document * | | |
| A | DU HUI ET AL: "Hybridization-based unquenching of DNA hairpins on Au surfaces: Prototypical molecular beacon biosensors" JOURNAL OF THE AMERICAN CHEMICAL SOCIETY, AMERICAN CHEMICAL SOCIETY, WASHINGTON, DC, US, vol. 125, no. 14, 9 April 2003 (2003-04-09), pages 4012-4013, XP002307240 ISSN: 0002-7863 * the whole document * | | TECHNICAL FIELDS SEARCHED (IPC) |
| A | LIU X ET AL: "Molecular beacons for DNA biosensors with micrometer to submicrometer dimensions" ANALYTICAL BIOCHEMISTRY, ACADEMIC PRESS, SAN DIEGO, CA, US, vol. 283, no. 1, 15 July 2000 (2000-07-15), pages 56-63, XP002229687 ISSN: 0003-2697 * the whole document * | | |

-/--

The present search report has been drawn up for all claims

| Place of search | Date of completion of the search | Examiner |
|---|---|---|
| The Hague | 20 September 2007 | Bruma, Anja |

EPO FORM 1503 03.82 (P04C01)

**European Patent Office**

**EUROPEAN SEARCH REPORT**

Application Number

EP 07 00 2203

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | US 2005/048546 A1 (PENN SHARRON [US] ET AL) 3 March 2005 (2005-03-03) * the whole document * ----- | | |
| A | LAKOWICZ J R: "Radiative decay engineering 5: metal-enhanced fluorescence and plasmon emission" ANALYTICAL BIOCHEMISTRY, ACADEMIC PRESS, NEW YORK, NY, US, vol. 337, no. 2, 15 February 2005 (2005-02-15), pages 171-194, XP004728751 ISSN: 0003-2697 * the whole document * ----- | | |
| E | WO 2007/095527 A (UNIV MARYLAND BIOTECH INST [US]; GEDDES CHRIS D [US]) 23 August 2007 (2007-08-23) * the whole document * ----- | 1-25 | |

TECHNICAL FIELDS SEARCHED (IPC)

The present search report has been drawn up for all claims

| Place of search | Date of completion of the search | Examiner |
|---|---|---|
| The Hague | 20 September 2007 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 00 2203

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2007

| Patent document cited in search report | | Publication date | Patent family member(s) | | Publication date |
|---|---|---|---|---|---|
| WO 2004059279 | A | 15-07-2004 | AU<br>EP | 2003296014 A1<br>1583948 A2 | 22-07-2004<br>12-10-2005 |
| WO 02064837 | A | 22-08-2002 | CA<br>EP<br>JP<br>MX | 2438129 A1<br>1360332 A1<br>2004524528 T<br>PA03007147 A | 22-08-2002<br>12-11-2003<br>12-08-2004<br>18-11-2003 |
| US 6812334 | B1 | 02-11-2004 | US<br>US<br>US<br>US<br>US<br>US<br>US<br>US<br>US<br>US<br>US<br>US<br>US<br>US<br>US<br>US<br>US<br>US<br>US<br>US<br>US<br>US<br>US | 2003059777 A1<br>2003049630 A1<br>2002146720 A1<br>2002164605 A1<br>2002182611 A1<br>2002155458 A1<br>2002137070 A1<br>2002127574 A1<br>2002137071 A1<br>2003049631 A1<br>2003143538 A1<br>2002155459 A1<br>2003054358 A1<br>7098320 B1<br>2002160381 A1<br>2002155461 A1<br>2002155462 A1<br>2003124528 A1<br>2002137072 A1<br>2003068622 A1<br>2004219520 A1<br>2003148282 A1<br>2002182613 A1<br>2003044805 A1 | 27-03-2003<br>13-03-2003<br>10-10-2002<br>07-11-2002<br>05-12-2002<br>24-10-2002<br>26-09-2002<br>12-09-2002<br>26-09-2002<br>13-03-2003<br>31-07-2003<br>24-10-2002<br>20-03-2003<br>29-08-2006<br>31-10-2002<br>24-10-2002<br>24-10-2002<br>03-07-2003<br>26-09-2002<br>10-04-2003<br>04-11-2004<br>07-08-2003<br>05-12-2002<br>06-03-2003 |
| US 2003040000 | A1 | 27-02-2003 | AU<br>CA<br>EP<br>JP<br>WO | 2002366432 A1<br>2456204 A1<br>1438438 A2<br>2005517428 T<br>03070876 A2 | 09-09-2003<br>28-08-2003<br>21-07-2004<br>16-06-2005<br>28-08-2003 |
| WO 2006052548 | A | 18-05-2006 | NONE | | |
| WO 2005098441 | A | 20-10-2005 | CN<br>EP<br>US | 1930475 A<br>1730532 A2<br>2005221507 A1 | 14-03-2007<br>13-12-2006<br>06-10-2005 |
| US 6579726 | B1 | 17-06-2003 | NONE | | |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 07 00 2203

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2007

| Patent document cited in search report | | Publication date | Patent family member(s) | | Publication date |
|---|---|---|---|---|---|
| WO 2005019812 | A | 03-03-2005 | EP<br>JP<br>US | 1658488 A1<br>2007503581 T<br>2006246460 A1 | 24-05-2006<br>22-02-2007<br>02-11-2006 |
| US 2005048546 | A1 | 03-03-2005 | NONE | | |
| WO 2007095527 | A | 23-08-2007 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006170538 A
- US 5424186 A
- JP 2004346177 A

- US 5925517 A
- US 20050048546 A1

**Non-patent literature cited in the description**

- Molecular-beacon-based array for sensitive DNA analysis. *Analytical Biochemistry,* 2003, vol. 331, 216
- *Analytical Biochemistry,* 2003, vol. 331, 216
- Single-mismatch detection using gold-quenched fluorescent oligonucleotides. *Nature Biotechnology,* 2001, vol. 19, 365
- Gold nanoparticle-assisted oligonucleotide immobilization for improved DNA detection. *IEE Proc. - Nanobiotechnol.,* 2005, vol. 152, 97
- NA hybridization assays using metal-enhanced fluorescence. *Biochemical and Biophysical Research Communications,* 2003, 306

- Radiative Decay Engineering: Biophysical and Biomedical Applications. *Analytical Biochemistry,* 2001, vol. 298, 1
- Bioconjugate Techniques. *Elsevier Science,* 1996, 140
- Interparticle Coupling Effects on Plasmon Resonances of Nanogold Particles. *Nano Letters,* 2003, vol. 3 (8), 1087-1090
- Optical Properties of Two Interacting Gold Nanoparticles. *Optics Communications,* 2003, vol. 220, 137-141
- Electrodynamics of noble metal nanoparticles and nanoparticle clusters. *Journal of Cluster Science,* 1999, vol. 10 (2

SEQUENCE LISTING

<110> Hitachi, Ltd.

<120> Biosensor element and method for manufacturing the same

<130> K600438

<150> JP2006-170538
<151> 2006-06-20

<160> 9

<170> PatentIn Ver. 2.1

<210> 1
<211> 30
<212> DNA
<213> Artificial Sequence

<220>
<223> Description of Artificial Sequence:Synthetic DNA

<400> 1
tccgcaaaaa aaaaaaaaaa aaaaagcgga                                    30

<210> 2
<211> 17
<212> DNA

<213> Artificial Sequence

<220>

<223> Description of Artificial Sequence:Synthetic DNA

<400> 2

agagatacat tgacctt                                                                 17

<210> 3

<211> 21

<212> DNA

<213> Artificial Sequence

<220>

<223> Description of Artificial Sequence:Synthetic DNA

<400> 3

cccttctcac tgttctctca t                                                            21

<210> 4

<211> 50

<212> DNA

<213> Artificial Sequence

<220>

<223> Description of Artificial Sequence:Synthetic DNA

<400> 4

agtcgagcgg tagcacagag agcttgctct cgggtgacga gcggcggacg          50

<210> 5

<211> 50

<212> DNA

<213> Artificial Sequence

<220>

<223> Description of Artificial Sequence:Synthetic DNA

<400> 5

cgtccgccgc tcgtcacccg agagcaagct ctctgtgcta ccgctcgact          50

<210> 6

<211> 18

<212> DNA

<213> Artificial Sequence

<220>

<223> Description of Artificial Sequence:Synthetic DNA

<400> 6

agtcgagcgg tagcacag          18

<210> 7

<211> 18

<212> DNA

<213> Artificial Sequence

<220>

<223> Description of Artificial Sequence:Synthetic DNA

<400> 7

ctgtgctacc gctcgact                                                    18

<210> 8

<211> 18

<212> DNA

<213> Artificial Sequence

<220>

<223> Description of Artificial Sequence:Synthetic DNA

<400> 8

aagtgagcat cattcact                                                    18

<210> 9

<211> 50

<212> DNA

<213> Artificial Sequence

<220>

<223> Description of Artificial Sequence:Synthetic DNA

<400> 9

```
tgagtttttt aacccgatga ttgtactgca acaagtgagc atcattcact                    50
```